(19) Europäisches Patentamt
European Patent Office
Office européen des brevets

(11) **EP 3 744 189 B1**

(12) **EUROPEAN PATENT SPECIFICATION**

(45) Date of publication and mention of the grant of the patent:
**28.02.2024 Bulletin 2024/09**

(21) Application number: **20176507.0**

(22) Date of filing: **26.05.2020**

(51) International Patent Classification (IPC):
*A24F 40/10* (2020.01)     *A24F 40/51* (2020.01)
*A24F 40/57* (2020.01)     *A61M 15/06* (2006.01)
*A61M 11/04* (2006.01)

(52) Cooperative Patent Classification (CPC):
**A61M 11/042; A24F 40/51; A24F 40/57;**
**A61M 15/06;** A24F 40/10; A61M 2016/0018;
A61M 2016/0027; A61M 2016/0039;
A61M 2205/123; A61M 2205/3317;
A61M 2205/3331; A61M 2205/3368;
A61M 2205/3389; A61M 2205/502; A61M 2205/581;

(Cont.)

(54) **AEROSOL INHALATION DEVICE**

AEROSOLINHALATIONSVORRICHTUNG

DISPOSITIF D'INHALATION D'AÉROSOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **31.05.2019 JP 2019102382**

(43) Date of publication of application:
**02.12.2020 Bulletin 2020/49**

(73) Proprietor: **Japan Tobacco Inc.**
**Tokyo 105-6927 (JP)**

(72) Inventors:
• **Mizuguchi, Kazuma**
  **Tokyo, 130-8603 (JP)**
• **Akao, Takeshi**
  **Tokyo, 130-8603 (JP)**
• **Aradachi, Takao**
  **Tokyo, 130-8603 (JP)**

(74) Representative: **Hoffmann Eitle**
**Patent- und Rechtsanwälte PartmbB**
**Arabellastraße 30**
**81925 München (DE)**

(56) References cited:
WO-A1-2017/144191    WO-A1-2017/144374
WO-A1-2018/001746    US-A1- 2019 089 180

(52) Cooperative Patent Classification (CPC): (Cont.)
A61M 2205/582; A61M 2205/583; A61M 2205/587;
A61M 2205/8206

**Description**

BACKGROUND OF THE INVENTION

Field of the Invention

The present invention relates to an aerosol inhalation device.

Description of the Related Art

In a general aerosol inhalation device such as an electronic cigarette, a heated tobacco product, or a nebulizer, which is used to generate an aerosol to be inhaled by a user, if the user performs inhalation when an aerosol source (to be also referred to as an "aerosol forming substrate" hereinafter) that changes to an aerosol by atomization is in shortage, a sufficient aerosol cannot be supplied to the user. Additionally, in a case of an electronic cigarette or a heated tobacco product, it is impossible to generate an aerosol having an intended flavor.

As a solution to this problem, PTL 1 discloses a technique of judging, based on the relationship between the temperature of a heating element and power applied to the heating element, a decrease in a liquid aerosol forming substrate heated by a heater (see the abstract and the like). PTL 2 discloses a technique of monitoring the operation of an electric heater and estimating the amount of a liquid aerosol forming substrate remaining in a liquid storage unit based on the monitored operation (see the abstract and the like). PTL 3 discloses a technique of obtaining a liquid level in a liquid storage portion based on the temperature measured value of a heater (see the abstract and the like).

WO2017144374A1 discloses an electrically operated aerosol-generating system (100, 300) for receiving a liquid aerosol-forming substrate (230, 430) and a main unit (101, 301) and a cartridge (200, 400) for an electrically operated aerosol-generating system (100, 300). The aerosol-generating system (100, 300) comprises: a liquid storage portion (210, 410) for holding a liquid aerosol-forming substrate (230, 430); aerosol-generating means (220, 420) arranged to receive liquid aerosol-forming substrate (230, 430) from the liquid storage portion (210, 410);one or more capillary wicks (250, 450) arranged to transfer liquid aerosol-forming substrate (230, 430) from the liquid storage portion (210, 410) to the aerosol-generating means (220, 420); a temperature sensor (270, 330) arranged to sense the temperature of liquid aerosol- forming substrate(230, 430) held in the liquid storage portion (210, 410); and electric circuitry (120, 320). The electric circuitry (120, 320) is configured to monitor the temperature of the liquid aerosol-forming substrate (230, 430) held in the liquid storage portion (210, 410) as sensed by the temperature sensor (270, 330)and determine depletion of liquid aerosol-forming substrate (230, 430) based on the temperature of the liquid aerosol-forming substrate (230, 430).

WO2017144191A1 provides an electrically operated aerosol-generating system (100) comprising a liquid storage portion (113) storing a liquid (115) from which aerosol may be generated, an electric heater (119), a capillary wick (117) positioned between the liquid (115) in the liquid storage portion (113) and the electric heater (119) and configured to convey liquid (115) from the liquid storage portion (113) to the electric heater (119) and electric circuitry (109) connected to the electric heater (119), the electric circuitry (109) configured to: activate the electric heater (119) for a vaporising period in response to a user input to vaporise liquid (115) in the capillary wick (117), a first predetermined time after the vaporising period activate the heater (119) for a second period, record a temperature measurement of the heater (119) during the second period, and determine a liquid level in the liquid storage portion (113) based on the temperature measurement.

WO2018001746A1 described an aerosol-generating system comprising: an electrically operated aerosol-generating element (102); a first electrochemical energy storage device (EESD, charging battery 106, first battery 126) configured to supply electrical power to the aerosol-generating element (102); and a EESD temperature control system comprising at least one temperature sensor (200, 210) positioned to sense a temperature of the first EESD (106, 126), and an electrical heater (140, 144) configured to heat the first EESD (106, 126), wherein the EESD temperature control system operates the electrical heater (140, 144) e.g. by means of microcontrollers (142, 146) dependent on an output from the at least one temperature sensor (200, 210).

However, it is difficult to correctly derive the temperature of the heater because physical characteristics change between individual heaters, and the temperature of the heater greatly varies in accordance with the use state of the aerosol inhalation device.

Patent Literatures

PTL 1: WO 2012/085203

PTL 2: WO 2012/085207
PTL 3: WO 2017/144191

SUMMARY OF THE INVENTION

The present invention has been made in consideration of the above-described points.

A problem to be solved by the present invention is to correctly acquire the temperature of the heater of an aerosol inhalation device and correctly estimate the remaining amount of an aerosol source.

The object of the invention is achieved by the subject-matter of the independent claim 1.

Advantageous embodiments are defined in the dependent claims. Further examples are provided for facilitating the understanding of the invention. According to an embodiment of the present invention, there is provided an aerosol inhalation device comprising a first sensor configured to output one of a value concerning an electrical resistance value of a load and the electrical resistance value, the load being configured to heat an aerosol source and having a correlation between a temperature and the electrical resistance value, a power supply, a second sensor comprising a thermistor configured to detect a temperature of the power supply, and a control unit configured to calculate the temperature of the load and/or judge whether the aerosol source is depleted based on an output value of the first sensor and an output value of the second sensor which are obtained before a start of power feed to the load for aerosol generation and an output value of the first sensor which is obtained after the start of power feed to the load for aerosol generation.

In the embodiment of the present invention, the aerosol inhalation device comprises a first unit including the load, and a second unit including the first sensor, the second sensor, the power supply and the control unit. The first unit is configured to be detachable from the second unit.

In the embodiment, the control unit is configured to acquire the output value of the second sensor before the output value of the first sensor and before the start of power feed to the load for aerosol generation.

In the embodiment, the control unit is configured to acquire a current output value of the second sensor, which is used in place of a previously acquired output value of the second sensor, before the start of power feed to the load for aerosol generation only if a condition to judge that a difference between the temperature of the load and the output value of the second sensor is less than a threshold is satisfied.

In the embodiment, the control unit is configured to acquire a current output value of the second sensor, which is used in place of a previously acquired output value of the second sensor, before the start of power feed to the load for aerosol generation only if a condition to judge that the temperature of the load and the output value of the second sensor are almost equal is satisfied.

In the embodiment, the control unit is configured to acquire a current output value of the second sensor, which is used in place of a previously acquired output value of the second sensor, before the start of power feed to the load for aerosol generation only if a condition to judge that the temperature of the load and the output value of the second sensor have a correlation is satisfied.

In the embodiment, the control unit is configured to acquire a current output value of the second sensor, which is used in place of a previously acquired output value of the second sensor, before the start of power feed to the load for aerosol generation only if a time elapsed from an end of preceding power feed to the load for aerosol generation is not less than a predetermined time.

In the embodiment, the control unit is configured to acquire the output value of the first sensor and the output value of the second sensor before the start of power feed to the load for aerosol generation when the first unit is attached to the second unit.

In the embodiment, the aerosol inhalation device further comprises a third sensor configured to detect inhalation by a user. The control unit is configured to acquire the output value of the first sensor and the output value of the second sensor before the start of power feed to the load for aerosol generation when the inhalation is detected by the third sensor.

In the embodiment, the control unit is configured to acquire, at a plurality of timings, the output value of the first sensor and the output value of the second sensor before the start of power feed to the load for aerosol generation.

In the embodiment, the control unit is configured to acquire, at least at a first timing and a second timing, the output value of the first sensor and the output value of the second sensor before the start of power feed to the load for aerosol generation, upon judging that the temperature of the load and the output value of the second sensor do not have a predetermined relationship, use the output value of the first sensor and the output value of the second sensor, which are acquired at the first timing, to calculate the temperature of the load and/or judge whether the aerosol source is depleted, and upon judging that the temperature of the load and the output value of the second sensor have a predetermined relationship, use the output value of the first sensor and the output value of the second sensor, which are acquired at the second timing, to calculate the temperature of the load and/or judge whether the aerosol source is depleted.

In the embodiment, the first timing is earlier than the second timing on a time base.

In the embodiment, the first timing is a timing at which the first unit is attached to the second unit.

In the embodiment, the aerosol inhalation device further comprises a third sensor configured to detect inhalation by a user. The second timing is a timing at which the inhalation is detected by the third sensor.

Further features of the present invention will become apparent from the following description of exemplary embodiments with reference to the attached drawings.

## BRIEF DESCRIPTION OF THE DRAWINGS

Fig. 1A is a schematic block diagram of the arrangement of an aerosol inhalation device according to an embodiment of the present invention;

Fig. 1B is a schematic block diagram of the arrangement of the aerosol inhalation device according to the embodiment of the present invention;

Fig. 2A shows an exemplary arrangement of a control device for the aerosol inhalation device according to the embodiment of the present invention;

Fig. 2B shows an exemplary arrangement of the control device for the aerosol inhalation device according to the embodiment of the present invention;

Fig. 3 shows a graph schematically showing a time-series change in the temperature of a load after the start of power feed to the load, and a temperature change of the load per predetermined time or predetermined supplied power;

Figs. 4A-1, 4A-2 and 4B show a flowchart of processing of calculating the temperature of the load and judging depletion or shortage of an aerosol source according to the embodiment of the present invention;

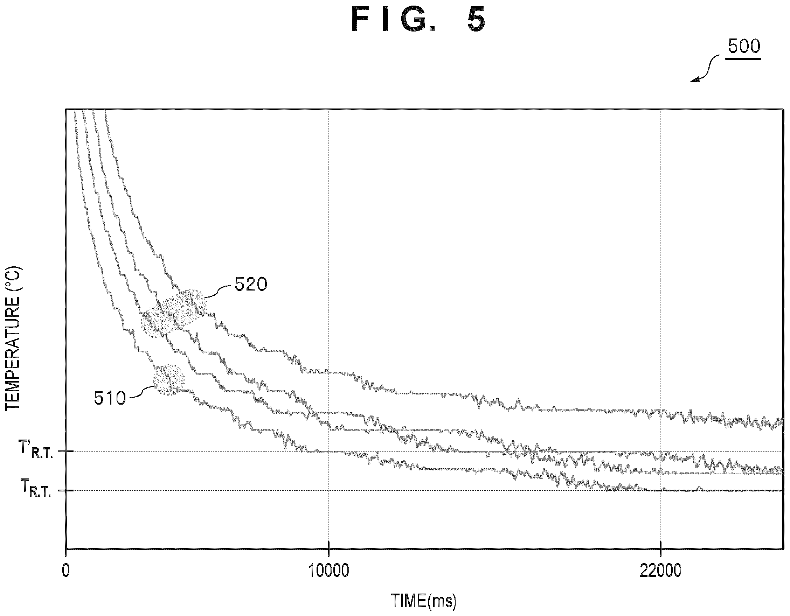

Fig. 5 is a graph showing a time-rate change in the temperature of the load after the stop of power feed to the load;

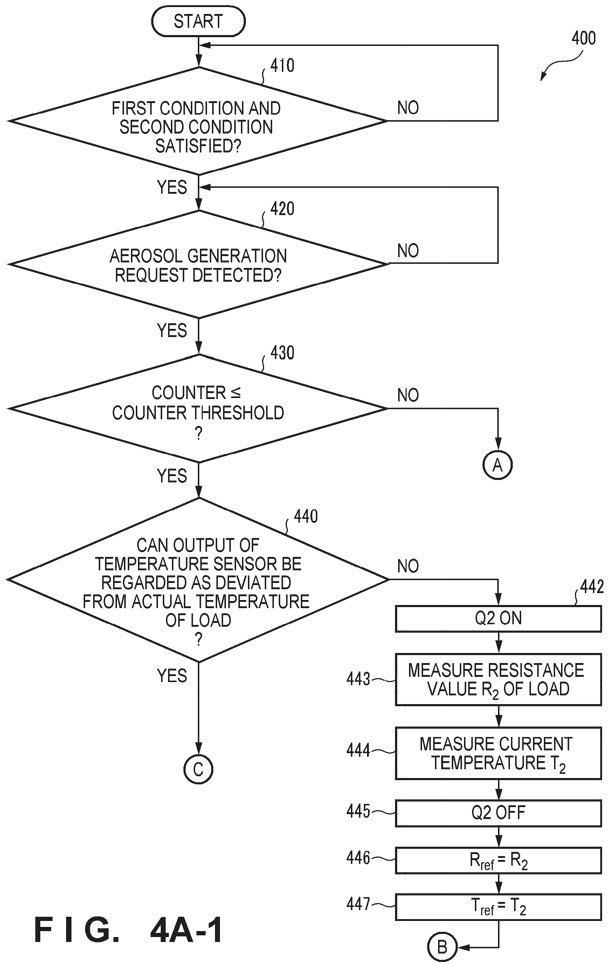

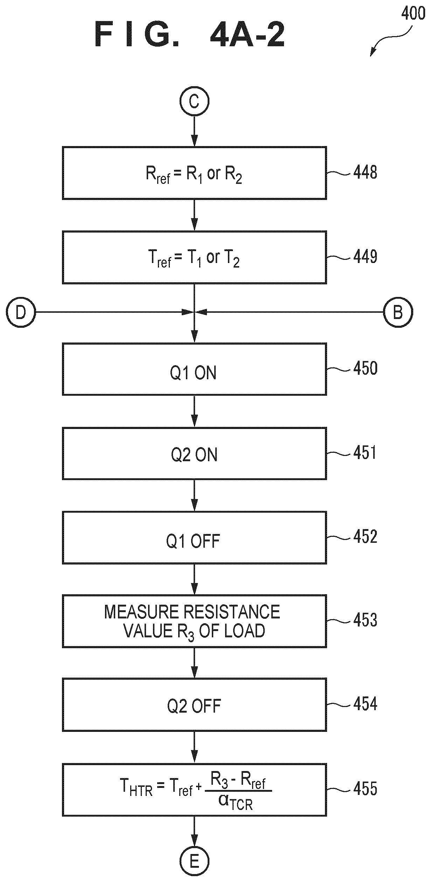

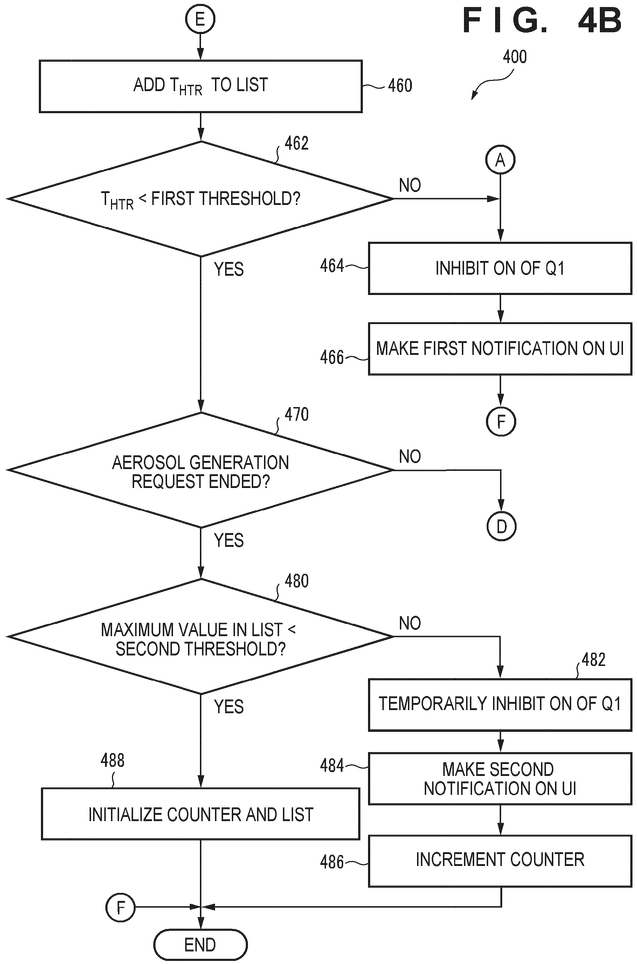

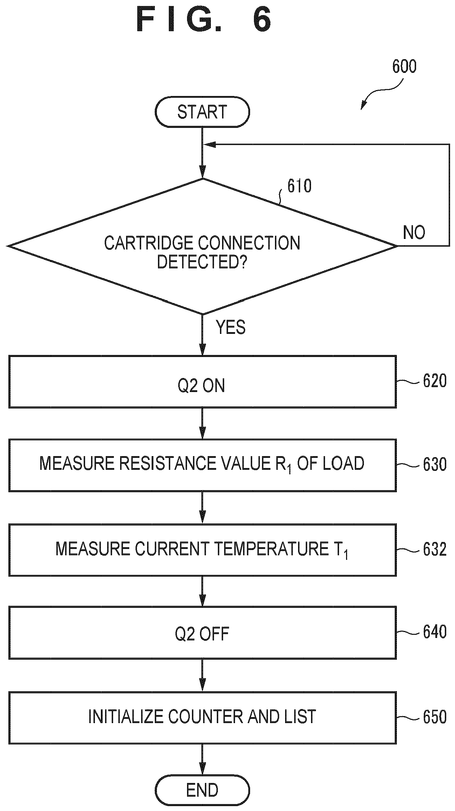

Fig. 6 is a flowchart of processing to be performed simultaneously with or in parallel to the processing shown in Figs. 4A and 4B;

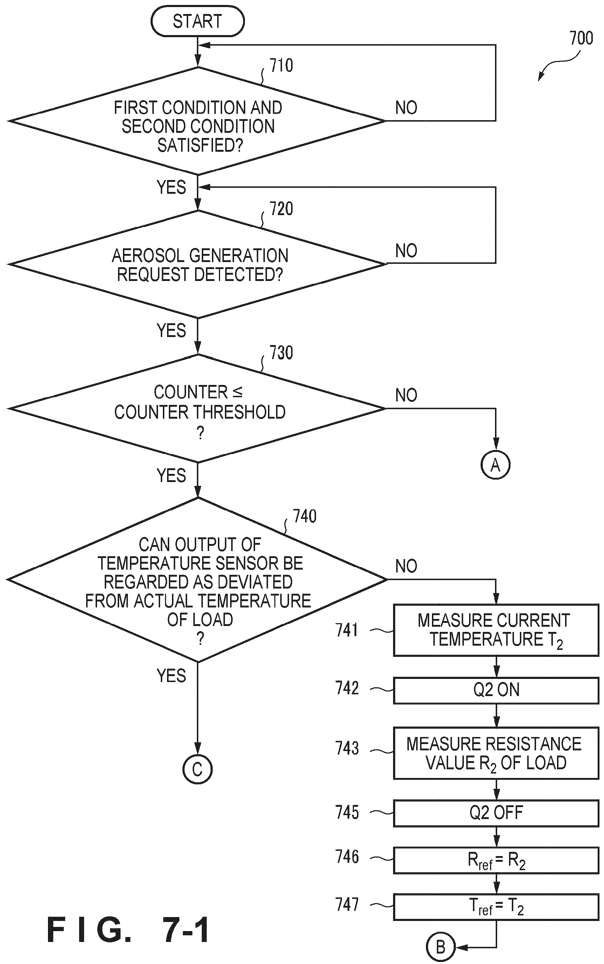

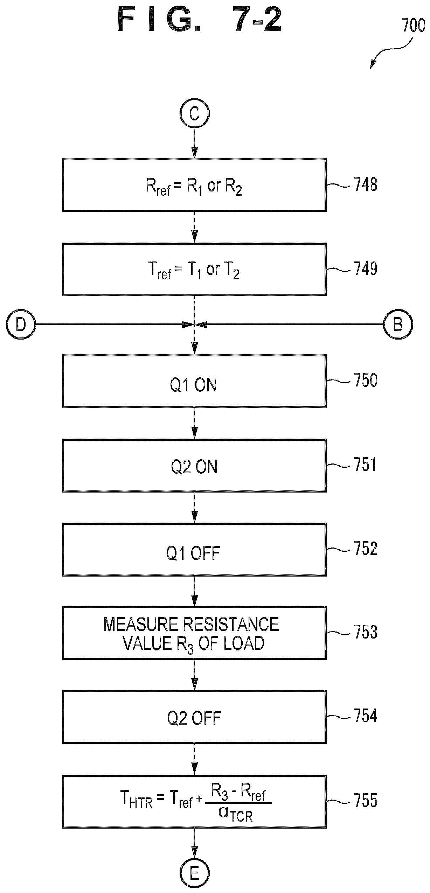

Figs. 7-1 and 7-2 show a flowchart of processing corresponding to Figs. 4A-1 and 4A-2 in an arrangement using a thermistor configured to detect the temperature of a power supply to acquire the temperature of the load according to the embodiment of the present invention; and

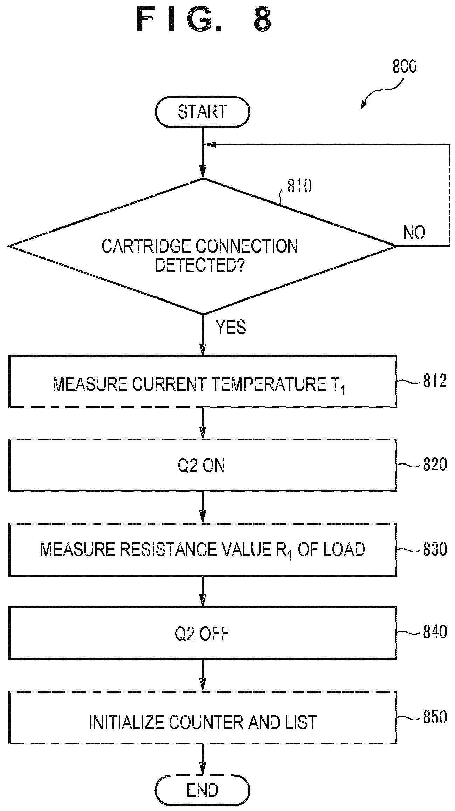

Fig. 8 is a flowchart of processing corresponding to Fig. 6 in the arrangement using a thermistor configured to detect the temperature of a power supply to measure the temperature of the load according to the embodiment of the present invention.

## DESCRIPTION OF THE EMBODIMENTS

An embodiment of the present invention will now be described in detail with reference to the accompanying drawings. Note that the embodiment of the present invention includes an electronic cigarette, a heated tobacco product, and a nebulizer, but is not limited to these. The embodiment of the present invention can include various aerosol inhalation devices configured to generate an aerosol to be inhaled by a user. In addition, the "aerosol inhalation device" according to this embodiment may also be called an aerosol generation device.

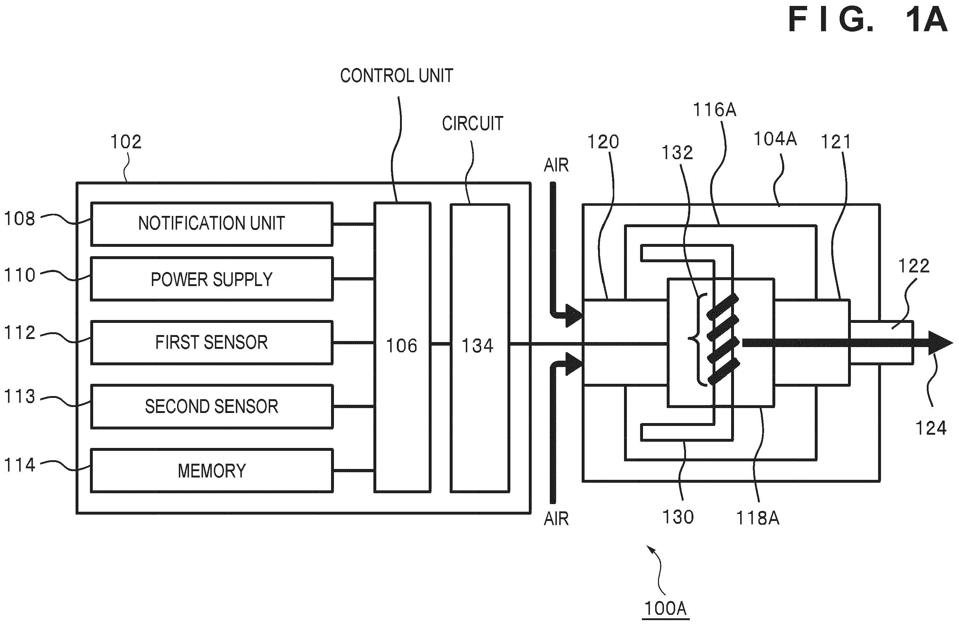

Fig. 1A is a schematic block diagram of the arrangement of an aerosol inhalation device 100 A according to an embodiment of the present invention. Fig. 1A schematically and conceptually shows components provided in the aerosol inhalation device 100A but does not show the strict arrangement, shapes, dimensions, positional relationship, and the like of the components and the aerosol inhalation device 100A.

As shown in Fig. 1A, the aerosol inhalation device 100A includes a second unit 102 (to be also referred to as a "main body 102" hereinafter), and a first unit 104A (to be also referred to as a "cartridge 104A" hereinafter). As shown in Fig. 1A, as an example, the main body 102 may include a control unit 106, a notification unit 108, a power supply 110, a first sensor 112, a second sensor 113, and a memory 114. The first sensor 112 may include a sensor configured to output a value concerning the electrical resistance value of a load configured to heat an aerosol source and having a correlation between a temperature and the electrical resistance value or the electrical resistance value. For example, the first sensor may be a voltage sensor, a current sensor, a temperature sensor, or the like. The second sensor 113 may be various sensors including a sensor configured to output a temperature. For example, the second sensor 113 may be a temperature sensor included in a thermistor configured to measure the temperature of the power supply 110, a temperature sensor included in a pressure sensor configured to detect inhalation by the user, or the like. The main body 102 may also include a flow velocity sensor, a flow rate sensor, and the like. The main body 102 may also include a circuit 134 to be described later. As an example, the cartridge 104A may include a storage unit 116A, an atomization unit 118A, an air intake channel 120, an aerosol channel 121, a mouthpiece portion 122, a holding portion 130, and a load 132. Some of the components included in the main body 102 may be included in the cartridge 104A. Some of the components included in the cartridge 104A may be included in the main body 102. The cartridge 104A may be configured to be detachable from the main body 102. Alternatively, all the components included in the main body 102 and the cartridge 104A may be included in a single housing in place of the main body 102 and the cartridge 104A.

The storage unit 116A may be formed as a tank to store an aerosol source. In this case, the aerosol source is, for example, a polyhydric alcohol such as glycerol or propylene, a liquid such as water, or a liquid mixture thereof. If the aerosol inhalation device 100 A is an electronic cigarette, the aerosol source in the storage unit 116A may contain a component that discharges a flavor component when heated. The holding portion 130 holds the aerosol source supplied from the storage unit 116A at a position where the load 132 can heat. For example, the holding portion 130 is made of a fibrous or porous material and holds an aerosol source as a liquid in gaps between fibers or in the pores of the porous material. As the above-described fibrous or porous material, for example, cotton, glass fiber, a tobacco raw material, or the like can be used. If the aerosol inhalation device 100 A is a medical inhalation device such as a nebulizer, the aerosol source may also contain a drug to be inhaled by a patient. As another example, the storage unit 116A may include a component capable of replenishing the consumed aerosol source. Alternatively, the storage unit 116A may be configured such that the storage unit 116A itself can be exchanged when the aerosol source is consumed. The aerosol source is not limited to a liquid and may be a solid. If the aerosol source is a solid, the storage unit 116A may be a hollow container.

The atomization unit 118A is configured to atomize the aerosol source and generate an aerosol. If an inhalation operation or another operation by the user is detected, the atomization unit 118A generates an aerosol. For example, the holding portion 130 is provided to connect the storage unit 116A and the atomization unit 118A. In this case, a part of the holding portion 130 communicates with the inside of the storage unit 116A and contacts the aerosol source. Another part of the holding portion 130 extends to the atomization unit 118A. Note that the other part of the holding portion 130 extending to the atomization unit 118A may be stored in the atomization unit 118A, or may communicate with the inside of the storage unit 116A via the atomization unit 118A. The aerosol source is carried from the storage unit 116A to the atomization unit 118A by the capillary effect of the holding portion 130. As an example, the atomization unit 118A includes a heater including the load 132 electrically connected to the power supply 110. The heater is arranged in contact with or in proximity of the holding portion 130. If an inhalation operation or another operation by the user is detected, the control unit 106 controls power supply to the heater of the atomization unit 118A and heats the aerosol source carried via the holding portion 130, thereby atomizing the aerosol source. The air intake channel 120 is connected to the atomization unit 118A, and the air intake channel 120 communicates with the outside of the aerosol inhalation device 100A. The aerosol generated by the atomization unit 118A is mixed with air taken via the air intake channel 120. The fluid mixture of the aerosol and air is sent to the aerosol channel 121, as indicated by an arrow 124. The aerosol channel 121 has a tubular structure configured to transport the fluid mixture of air and the aerosol generated by the atomization unit 118A to the mouthpiece portion 122.

The mouthpiece portion 122 is located at the end of the aerosol channel 121 and configured to open the aerosol channel 121 to the outside of the aerosol inhalation device 100A. The user holds the mouthpiece portion 122 in the mouth and inhales, thereby taking the air containing the aerosol into the oral cavity.

The notification unit 108 may include a light emitting element such as an LED, a display, a speaker, a vibrator, and the like. The notification unit 108 is configured to make some notification to the user as needed by light emission, display, utterance, vibration, or the like.

Note that the cartridge 104A can be formed as an outer tube, and one or both of the air intake channel 120 and the aerosol channel 121 can be formed as an inner tube arranged in the outer tube. The load 132 can be arranged in the air intake channel 120 or the aerosol channel 121 that is the inner tube. The storage unit 116A can be arranged or formed between the cartridge 104A that is the outer tube and the air intake channel 120 or the aerosol channel 121 that is the inner tube.

The power supply 110 supplies power to the components such as the notification unit 108, the first sensor 112, the second sensor 113, the memory 114, the load 132, and the circuit 134 in the aerosol inhalation device 100A. The power supply 110 may be a primary battery or a secondary battery that can be charged by connecting to an external power supply via a predetermined port (not shown) of the aerosol inhalation device 100A. Only the power supply 110 may be detachable from the main body 102 or the aerosol inhalation device 100A, or may be exchangeable with a new power supply 110. In addition, the power supply 110 may be exchangeable with a new power supply 110 by exchanging the whole main body 102 with a new main body 102. As an example, the power supply 110 may be formed by a lithium ion secondary battery, a nickel hydrogen secondary battery, a lithium ion capacitor, or the like. The power supply 110 that is the secondary battery may include a temperature sensor configured to detect the temperature of the battery. In the present invention, it can be interpreted that the second sensor 113 can include such a temperature sensor.

The first sensor 112 may include one or a plurality of sensors used to acquire the value of a voltage applied to the whole or a specific part of the circuit 134, the value of a current flowing to the whole or a specific part of the circuit 134, a value associated with the electrical resistance value of the load 132 or a value associated with the temperature, and the like. The first sensor 112 may be incorporated in the circuit 134. The function of the first sensor 112 may be incorporated in the control unit 106. The second sensor 113 may include one or a plurality of sensors used to acquire a value associated with the temperature in a place in the aerosol inhalation device 100A, and the like. The second sensor 113 may be included in a pressure sensor that detects a variation in the pressure in one or both of the air intake channel 120 and the aerosol channel 121. The aerosol inhalation device 100A may also include at least one of a flow velocity sensor that detects a flow velocity, and a flow rate sensor that detects a flow rate. The aerosol inhalation device 100A may also include a weight sensor that detects the weight of a component such as the storage unit 116A. The aerosol inhalation device 100A may also be configured to count the number of puffs by the user who uses the aerosol inhalation device 100A. The aerosol inhalation device 100A may also be configured to integrate the time of energization to the atomization unit 118A. The aerosol inhalation device 100A may also include a sensor configured to detect the liquid level in the storage unit 116A. The aerosol inhalation device 100A may also include a sensor configured to obtain or detect the SOC (State Of Charge), current integrated value, voltage, and the like of the power supply 110. The SOC may be obtained by the current integration method (coulomb counting method), the SOC-OCV (Open Circuit Voltage) method, or the like. The second sensor 113 may also include a temperature sensor in the power supply 110. The aerosol inhalation device 100A may also be configured to be able to detect an operation for an operation button that can be operated by the user.

The control unit 106 can be an electronic circuit module formed as a microprocessor or a microcomputer, for example, an MPC. The control unit 106 may be configured to control the operation of the aerosol inhalation device 100A in accordance with computer executable instructions stored in the memory 114. The memory 114 is a storage medium such as a ROM, a RAM, or a flash memory. In addition to the computer executable instructions as described above, the memory 114 may store setting data necessary for control of the aerosol inhalation device 100A. For example, the memory 114 may store various data such as a control method (a form such as light emission, utterance, or vibration) of the notification unit 108, values obtained and/or detected by the first sensor 112 and the second sensor 113, and the heating history of the atomization unit 118A. The control unit 106 reads out data from the memory 114 as needed and uses it to control the aerosol inhalation device 100A, and stores data in the memory 114 as needed.

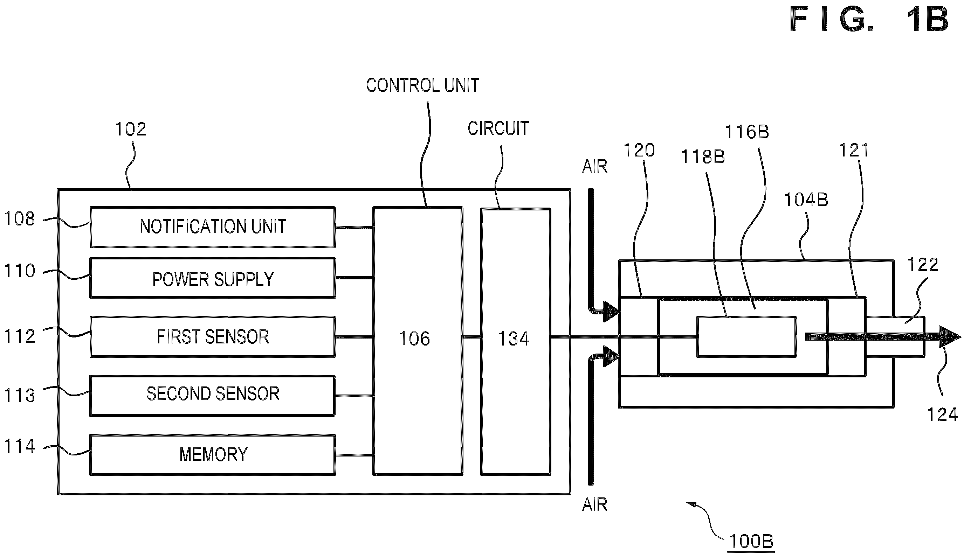

Fig. 1B is a schematic block diagram of the arrangement of an aerosol inhalation device 100B according to the embodiment of the present invention.

As shown in Fig. 1B, the aerosol inhalation device 100B has an arrangement similar to the aerosol inhalation device 100A shown in Fig. 1A. However, the arrangement of a first unit 104B (to be referred to as an "aerosol generating article 104B" or "stick 104B" hereinafter) is different from the arrangement of the first unit 104A. As an example, the aerosol generating article 104B may include an aerosol base material 116B, an atomization unit 118B, the air intake channel 120, the aerosol channel 121, and the mouthpiece portion 122. Some of the components included in the main body 102 may be included in the aerosol generating article 104B. Some of the components included in the aerosol generating article 104B may be included in the main body 102. The aerosol generating article 104B may be insertable/removable into/from the main body 102. Alternatively, all the components included in the main body 102 and the aerosol generating article 104B may be included in a single housing in place of the main body 102 and the aerosol generating article 104B.

The aerosol base material 116B may be formed as a solid carrying an aerosol source. As in the storage unit 116A shown in Fig. 1A, the aerosol source may be, for example, a polyhydric alcohol such as glycerol or propylene, a liquid such as water, or a liquid mixture thereof. The aerosol source in the aerosol base material 116B may contain a tobacco raw material or an extract derived from a tobacco raw material, which discharges a flavor component when heated. Note that the aerosol base material 116B itself may be made of a tobacco raw material. If the aerosol inhalation device 100B is a medical inhalation device such as a nebulizer, the aerosol source may also contain a drug to be inhaled by a patient. The aerosol base material 116B may be configured such that the aerosol base material 116B itself can be exchanged when the aerosol source is consumed. The aerosol source is not limited to a liquid and may be a solid.

The atomization unit 118B is configured to atomize the aerosol source and generate an aerosol. If an inhalation operation or another operation by the user is detected, the atomization unit 118B generates an aerosol. The atomization unit 118B includes a heater (not shown) including a load electrically connected to the power supply 110. If an inhalation operation or another operation by the user is detected, the control unit 106 controls power supply to the heater of the atomization unit 118B and heats the aerosol source carried in the aerosol base material 116B, thereby atomizing the aerosol source. The air intake channel 120 is connected to the atomization unit 118B, and the air intake channel 120 communicates with the outside of the aerosol inhalation device 100B. The aerosol generated by the atomization unit 118B is mixed with air taken via the air intake channel 120. The fluid mixture of the aerosol and air is sent to the aerosol channel 121, as indicated by the arrow 124. The aerosol channel 121 has a tubular structure configured to transport the fluid mixture of air and the aerosol generated by the atomization unit 118B to the mouthpiece portion 122.

The control unit 106 is configured to control the aerosol inhalation devices 100A and 100B (to be collectively referred to as the "aerosol inhalation device 100" hereinafter) according to the embodiment of the present invention by various methods.

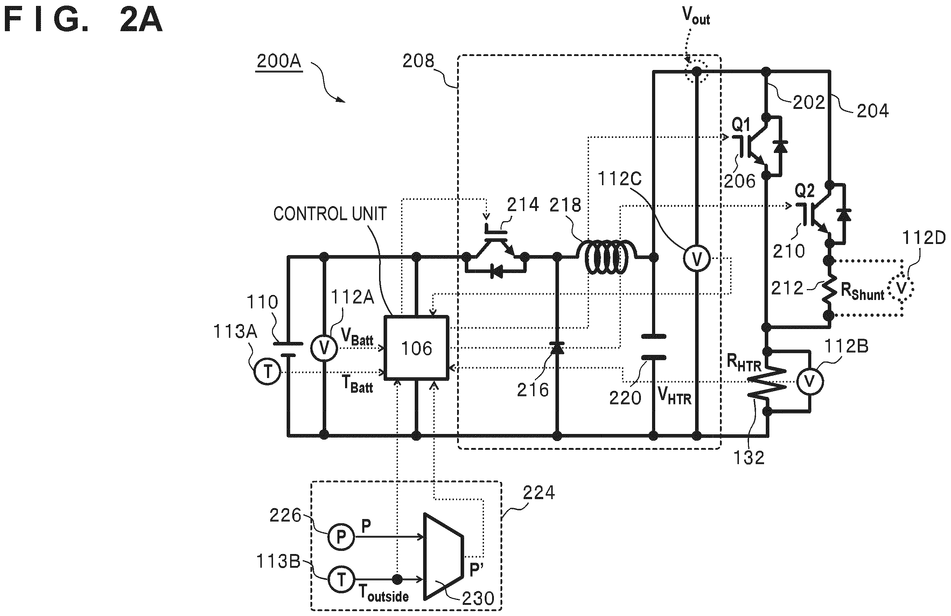

Fig. 2A is a circuit diagram showing an exemplary arrangement of a control device for the aerosol inhalation device 100 according to the embodiment of the present invention.

A control device 200A shown in Fig. 2A includes the power supply 110, the control unit 106, first sensors 112A to 112D (to be collectively referred to as the "first sensor 112" hereinafter), second sensors 113Aand/or 113B (to be collectively referred to as the "second sensor 113" hereinafter), the load 132 (to be also referred to as a "heater resistor" hereinafter), a first circuit 202, a second circuit 204, a switch Q1 including a first field effect transistor (FET) 206, a conversion unit 208, a switch Q2 including a second FET 210, and a resistor 212 (to be also referred to as a "first shunt resistor" hereinafter).

In an example, the first sensor 112 may be a voltage sensor. In particular, the first sensor 112B may be used to measure a value concerning the electrical resistance value of the load 132 or the electrical resistance value. According to the claimed invention, the second sensor 113A is a thermistor that measures the temperature of the power supply 110. The control device 200A may include a pressure sensor 224 that detects inhalation by the user. The pressure sensor 224 may include the second sensor 113B that is a temperature sensor configured to measure an outside air temperature $T_{outside}$, an absolute pressure sensor 226 that measures an absolute pressure P, and a calibration unit 230. The pressure sensor 224 calibrates a pressure measured by the absolute pressure sensor 226 using a temperature measured by the second sensor 113B, thereby correctly acquiring a pressure P' in the aerosol inhalation device 100. The calibration unit 230 may be formed by a multiplexer. The control device 200A can include at least one of the second sensors 113A and 113B. If a certain time has elapsed from the stop of power feed to the load 132 for aerosol generation, the temperature of the power supply 110 and the temperature of the load 132 are the outside air temperature or a value close to the outside air temperature. If it is considered that the deviation between the temperature of the power supply 110 and the temperature of the load 132 is sufficiently small, the temperature measured by the second sensor 113A can be regarded as the temperature of the load 132. In addition, the second sensor 113B measures the outside air temperature. If a certain time has elapsed from the stop of power feed to the load 132 for aerosol generation, the temperature of the load 132 is the outside air temperature or a value close to the outside air temperature. In this case, the temperature measured by the second sensor 113B can be regarded as the temperature of the load 132.

As described above, according to this embodiment, the temperature sensor included in the pressure sensor 224 that detects inhalation by the user can be used to acquire the temperature of the load 132. Alternatively, according to this embodiment, the temperature sensor (thermistor) configured to detect a temperature $T_{Batt}$ of the power supply 110 can be used to acquire the temperature of the load 132. According to such a feature, since a separate sensor need not be prepared to acquire the temperature of the load 132, the cost of the aerosol inhalation device 100 (in particular, the cartridge 104A) can be suppressed low.

The electrical resistance value and the temperature of the load 132 have a correlation, and the electrical resistance value changes in accordance with the temperature. In other words, the load 132 can include a PTC heater. The first shunt resistor 212 is electrically connected in series with the load 132 and has a known electrical resistance value. The electrical resistance value of the first shunt resistor 212 can be almost or completely invariable with respect to the temperature. The first shunt resistor 212 has an electrical resistance value sufficiently larger than that of the load 132. The first sensors 112C and 112D may be omitted in accordance with the embodiment. The first FET 206 included in the switch Q1 and the second FET 210 included in the switch Q2 each play a role of a switch that opens/closes an electrical circuit. It would be obvious for those skilled in the art that as the switch, not only an FET but also various elements such as an IGBT and a contactor can be used as the switches Q1 and Q2. In addition, the switches Q1 and Q2 preferably have the same characteristic, but may not. Hence, the FETs, IGBTs, contactors, or the like used as the switches Q1 and Q2 preferably have the same characteristic, but may not. Note that if elements having the same characteristic are employed as the switches Q1 and Q2, the procurement cost for each of the switches Q1 and Q2 can be reduced. This makes it possible to manufacture the control device 200A at a lower cost.

The conversion unit 208 can be, for example, a switching converter, and can include an FET 214, a diode 216, an inductor 218, and a capacitor 220. The control unit 106 may control the conversion unit 208 such that the conversion unit 208 converts the output voltage of the power supply 110, and the converted output voltage is applied to the whole circuit. Here, the conversion unit 208 is preferably configured to output a predetermined voltage under the control of the control unit 106 during the time when at least the switch Q2 is in an ON state. In addition, the conversion unit 208 may be configured to output a predetermined voltage under the control of the control unit 106 during the time when the switch Q1 is in an ON state as well. Note that in these cases, the voltage output by the conversion unit 208 need not strictly be constant. If the target voltage of the conversion unit 208 is maintained constant for a predetermined period, it can be said that the conversion unit 208 is configured to output a predetermined voltage. Note that the predetermined voltage output by the conversion unit 208 under the control of the control unit 106 during the ON state of the switch Q1 and the predetermined voltage output by the conversion unit 208 under the control of the control unit 106 during the ON state of the switch Q2 may be equal or different. If these are different, the predetermined voltage output by the conversion unit 208 under the control of the control unit 106 during the ON state of the switch Q1 may be higher or lower than the predetermined voltage output by the conversion unit 208 under the control of the control unit 106 during the ON state of the switch Q2. According to this arrangement, since the voltages and other parameters are stable, the estimation accuracy of the temperature of the load 132 and the remaining aerosol amount estimation accuracy improve. Furthermore, when a switching regulator is used as the conversion unit 208, a loss generated when a voltage input to the conversion unit 208 is converted into a predetermined voltage can be made small. This can generate a larger amount of aerosol by one charge while improving the remaining aerosol amount detection accuracy. The conversion unit 208 may be configured to directly apply the output voltage of the power supply 110 to the first circuit under the control of the control unit 106 during the time when only the switch Q1 is in the ON state. This form may be implemented by the control unit 106 controlling the switching converter in a direct connection mode in which the switching operation stops. Note that the conversion unit 208 is not an essential component but may be omitted. The conversion unit 208 may be of a step-down type shown in Fig. 2A, or may be of a boost type or a step-down/boost type.

Note that the control of the conversion unit 208 may be done by another control unit other than the control unit 106. The other control unit may be provided in the conversion unit 208. In this case, a value detected by the sensor 112C is input at least to the other control unit. Note that in this case as well, a value detected by the sensor 112C may be input to the control unit 106.

The circuit 134 shown in Figs. 1A and 1B electrically connects the power supply 110 and the load 132, and can include the first circuit 202 and the second circuit 204. The first circuit 202 and the second circuit 204 are electrically connected in parallel with the power supply 110 and the load 132. The first circuit 202 can include the switch Q1. The second circuit 204 can include the switch Q2 and the resistor 212 (and the first sensor 112D as an option). The first circuit 202 may have a resistance value smaller than that of the second circuit 204. In this example, the first sensors 112B and 112D are voltage sensors, and are each configured to detect the potential difference (to be also referred to as a "voltage" or "voltage value" hereinafter) across the load 132 and the resistor 212. However, the arrangement of the first sensor 112 is not limited to this. For example, the first sensor 112 may be a current sensor and may detect the value of a current flowing to one or both of the load 132 and the resistor 212.

As indicated by dotted arrows in Fig. 2A, the control unit 106 can control the switch Q1, the switch Q2, and the like, and can acquire a value detected by the first sensor 112. The control unit 106 may be configured to cause the first circuit 202 to function by switching the switch Q1 from an OFF state to an ON state and cause the second circuit 204 to function by switching the switch Q2 from an OFF state to an ON state. The control unit 106 may be configured to cause the first circuit 202 and the second circuit 204 to alternately function by alternately switching the switches Q1 and Q2.

The first circuit 202 is mainly used to atomize the aerosol source. When the switch Q1 is switched to the ON state, and the first circuit 202 functions, power is supplied to the heater (that is, the load 132 in the heater), and the load 132 is heated. By heating the load 132, the aerosol source held by the holding portion 130 in the atomization unit 118A(in the aerosol inhalation device 100B shown in Fig. 1B, the aerosol source carried by the aerosol base material 116B) is atomized, and an aerosol is generated.

The second circuit 204 is used to acquire the value of a voltage applied to the load 132, the value of a current flowing to the load 132, the value of a voltage applied to the resistor 212, the value of a current flowing to the resistor 212, and the like. The acquired value of the voltage or current can be used to acquire the resistance value of the load 132. A case in which the switch Q1 is in the Off state, and the first circuit 202 is not functioning, and the switch Q2 is in the ON state, and the second circuit 204 is functioning will be examined. In this case, since the current flows to the switch Q2, the first shunt resistor 212, and the load 132, a resistance value $R_{HTR}$ of the load 132 can be acquired by calculation using, for example,

$$R_{HTR} = \frac{V_{HTR}}{V_{out} - V_{HTR}} \cdot R_{shunt}$$
$$= \frac{V_{out} - V_{shunt}}{V_{shunt}} \cdot R_{shunt}$$
$$= \frac{V_{out}}{I_{HTR}} - R_{shunt} \qquad (1)$$
$$= \frac{V_{HTR}}{I_{HTR}}$$

where $V_{out}$ is a voltage that can be detected by the first sensor 112C or a predetermined target voltage to be output by the conversion unit 208, and represents a voltage applied to the whole of the first circuit 202 and the second circuit 204. Note that if the conversion unit 208 is not used, the voltage $V_{out}$ may be a voltage $V_{Batt}$ that can be detected by the first sensor 112A. $V_{HTR}$ represents a voltage applied to the load 132, which can be detected by the first sensor 112B, and $V_{shunt}$ represents a voltage applied to the first shunt resistor 212, which can be detected by the first sensor 112D. $I_{HTR}$ represents a current flowing to the load 132 (same as the current flowing to the first shunt resistor 212 in this case), which can be detected by a sensor (for example, a Hall element) or the like (not shown). $R_{shunt}$ represents a known resistance value of the first shunt resistor 212, which can be decided in advance.

Note that the resistance value of the load 132 can be obtained using at least equation (1) regardless of whether the switch Q2 is functioning even if the switch Q1 is in the ON state. In the embodiment of the present invention, this means that the output value of the first sensor 112 acquired when the switch Q1 is in the ON state can be used, or a circuit in which the second circuit 204 does not exist can be used. Note that the above-described method is merely an example, and the resistance value of the load 132 can be obtained by an arbitrary method.

The acquired resistance value of the load 132 can be used to acquire the temperature of the load 132. More specifically, if the load 132 has a positive or negative temperature coefficient characteristic (the positive temperature coefficient characteristic is sometimes called a "PTC characteristic") representing that the resistance value changes in accordance with the temperature, a temperature $T_{HTR}$ of the load 132 can be estimated based on the relationship (that is, correlation) between the resistance value and the temperature of the load 132, which is known in advance, and the resistance value $R_{HTR}$ of the load 132, which is obtained by equation (1). More specifically, the resistance value $R_{HTR}$ and the temperature $T_{HTR}$ of the load 132 have a relationship represented by

$$R_{HTR} = R_{ref} + \left(T_{HTR} - T_{ref}\right) \cdot \alpha_{TCR} \qquad (2)$$

Hence,

$$T_{HTR} = \frac{R_{HTR} - R_{ref}}{\alpha_{TCR}} + T_{ref} \qquad (3)$$

where $T_{ref}$ is a predetermined reference temperature, $R_{ref}$ is a reference resistance value, and $\alpha_{TCR}$ is a known constant depending on the material of the load 132. To correctly obtain the temperature $T_{HTR}$ of the load 132, the reference resistance value $R_{ref}$ needs to equal the resistance value of the load 132 at the reference temperature $T_{ref}$. That is, when the load 132 is set to the desired reference temperature $T_{ref}$, and the resistance value of the load 132 at that point of time is acquired as the reference resistance value $R_{ref}$, the unknown temperature $T_{HTR}$ of the load 132 at an arbitrary point of time can be acquired by calculation using equation (3) by giving the resistance value $R_{HTR}$ of the load 132 obtained by equation (1) at that point of time.

The resistance value between the terminals of the load 132 when the cartridge 104A is attached to the main body 102 is a value according to the resistance value of the load 132 included in the cartridge 104A. On the other hand, the resistance value between the terminals when the cartridge 104a is detached from the main body 102 exhibits an infinite or extremely large value. This is because when the cartridge 104A is detached from the main body 102, the terminals are insulated by air.

Hence, exchange of the cartridge 104A can be detected by, for example, detecting that the resistance value between the terminals exceeds a predetermined value larger than the value according to the resistance value of the load 132 and then falls below the predetermined value again.

In addition, the electronic circuit of the main body 102 can be configured such that when a predetermined voltage is applied, the potential difference (voltage) between the terminals when the cartridge 104A is attached to the main body 102 has a value according to the resistance value of the load 132 included in the cartridge 104A, and the potential difference (voltage) between the terminals when the cartridge 104A is detached from the main body 102 becomes larger than the value according to the resistance value of the load 132.

Hence, exchange of the cartridge 104A can be detected by, for example, applying a predetermined voltage to the electronic circuit of the main body 102 and detecting that the potential difference (voltage) between the terminals exceeds a predetermined value larger than the value according to the resistance value of the load 132 and then falls below the predetermined value again.

The aerosol inhalation device 100 according to the embodiment of the present invention judges the occurrence of depletion or shortage of the aerosol source. In the present invention, "depletion" of the aerosol source means a state in which the remaining amount of the aerosol source is zero or almost zero. In the present invention, "shortage" of the aerosol source means a state in which the remaining amount of the aerosol source is not sufficient but not depleted. Alternatively, it may mean a state in which the remaining amount of the aerosol source is sufficient for instantaneous aerosol generation but insufficient for continuous aerosol generation. Alternatively, it may mean a state in which the remaining amount of the aerosol source is not sufficient for generating an aerosol with a sufficient flavor.

If the aerosol source is in a saturation state in the aerosol base material 116B or the holding portion 130, the temperature of the load 132 enters a steady state at the boiling point of the aerosol source or a temperature at which aerosol generation occurs due to evaporation of the aerosol source (to be referred to as a "boiling point or the like" hereinafter). This phenomenon can be understood from the fact that at these temperatures as the boundary, heat generated in the load 132 by power supplied from the power supply 110 is used not to heat the aerosol source but to evaporate the aerosol source or generate the aerosol. Here, even in a case in which the aerosol source is not in the saturation state in the aerosol base material 116B or the holding portion 130, but the remaining amount is a predetermined amount or more, the temperature of the load 132 enters the steady state at the boiling point or the like. In the present invention, that the remaining amount of the aerosol source in the aerosol base material 116B or the holding portion 130 is "sufficient" means a state in which the remaining amount of the aerosol source in the aerosol base material 116B or the holding portion 130 is the predetermined amount or more, or the remaining amount of the aerosol source in the aerosol base material 116B or the holding portion 130 is in such a state (including the saturation state) that the temperature of the load 132 enters the steady state at the boiling point or the like. Note that in the latter case, the detailed remaining amount of the aerosol source in the aerosol base material 116B or the holding portion 130 need not be specified. The boiling point of the aerosol source and the temperature at which aerosol generation occurs match if the aerosol source is a liquid of a single composition. On the other hand, if the aerosol source is a liquid mixture, a theoretical boiling point of the liquid mixture, which is obtained by the Raoult's law, may be regarded as the temperature at which aerosol generation occurs. Alternatively, the temperature at which aerosol generation occurs due to boiling of the aerosol source may be obtained by experiments.

Furthermore, if the remaining amount of the remaining amount in the storage unit 116A is smaller than a predetermined amount, in principle, the aerosol source is not supplied any more from the storage unit 116A to the holding portion 130 (in some cases, a very small amount of aerosol source is supplied, or supply is done to some extent by tilting or shaking the aerosol inhalation device 100). In the present invention, that the remaining amount of the aerosol source is sufficient concerning the storage unit 116A means a state in which the remaining amount of the aerosol source in the storage unit 116A is the predetermined amount or more, or supply can be done to set the aerosol source in the holding portion 130 in the saturation state or set the remaining amount of the aerosol source to the predetermined amount or more. Note that in the latter case, since it can be estimated or judged that the remaining amount of the aerosol source in the storage unit 116A is sufficient because the temperature of the load 132 enters the steady state at the boiling point or the like, the detailed remaining amount of the aerosol source in the storage unit 116A need not be specified. Additionally, in this case, if the remaining amount of the aerosol source in the holding portion 130 is not sufficient (that is, short or depleted), it can be estimated or judged that the remaining amount of the aerosol source in the storage unit 116A is not sufficient (that is, short or depleted).

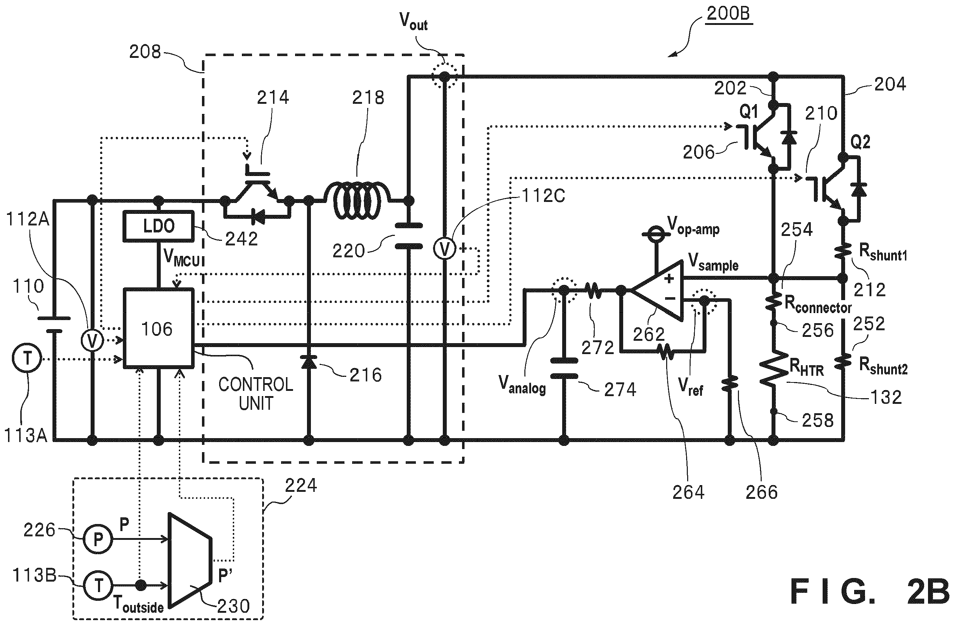

Fig. 2B is a circuit diagram showing an exemplary arrangement of a control device for the aerosol inhalation device 100 according to the embodiment of the present invention.

In addition to the arrangement of the control device 200A shown in Fig. 2A, a control device 200B includes a resistor 254 (its electrical resistance value is represented by $R_{CONNECTOR}$) electrically connected to the load 132, a connection terminal 256 and a connection terminal 258 of the load 132 to circuits, a linear regulator such as an LDO 242, a second shunt resistor 252, an operational amplifier 262, a resistor 264 and a resistor 266 which are electrically connected to the inverting input terminal of the operational amplifier 262, a resistor 272 electrically connected to the output terminal of the operational amplifier 262, and a capacitor 274 electrically connected to the resistor 272. In Fig. 2B, the electrical resistance values of the first shunt resistor 212 and the second shunt resistor are represented by $R_{shunt1}$ and $R_{shunt2}$, respectively.

$V_{sample}$ corresponds to a voltage applied to the noninverting input terminal of the operational amplifier 262. $V_{ref}$ corresponds to a voltage applied to the inverting input terminal of the operational amplifier 262. $V_{analog}$ corresponds to a voltage according to the voltage of the output terminal of the operational amplifier 262, which is a voltage applied to the control unit 106. $V_{op-amp}$ corresponds to the power supply voltage of the operational amplifier 262. $V_{MCU}$ corresponds to the output voltage of the LDO 242, which is a voltage applied to the power supply terminal of the control unit 106, that is, the power supply voltage of the control unit 106.

The load 132 is detachably electrically connected to the circuits of the control device 200B via the connection terminal 256 and the connection terminal 258. The load 132 may be included in the control device 200B or not. The resistor 254 represents the connection resistance of the load 132.

The first shunt resistor 212 is electrically connected in series with the load 132, and has the known electrical resistance value $R_{shunt1}$. The electrical resistance value $R_{shunt1}$ of the first shunt resistor 212 can be almost or completely invariable with respect to the temperature. The first shunt resistor 212 has an electrical resistance value larger than that of the load 132. The second shunt resistor 252 can have the same characteristic as the first shunt resistor 212, but is not limited to this.

A linear regulator such as the LDO 242 is electrically connected to the power supply terminal of the control unit 106, and generates the voltage $V_{MCU}$ to drive the control unit 106.

The voltage $V_{MCU}$ is a voltage used to drive the control unit 106 and can therefore be a relatively low voltage. On the other hand, the voltage $V_{out}$ is associated with a voltage applied to the load 132, and is preferably a relatively high voltage to improve the atomization efficiency. Hence, in general, the voltage $V_{out}$ is higher than the voltage $V_{MCU}$.

The operational amplifier 262 is used to form a voltage sensor that forms a part of the sensor 112. In the control device 200B, the operational amplifier 262 forms a part of an amplification circuit. Hence, the voltage $V_{analog}$ according to the voltage $V_{sample}$ (exactly, according to the difference between the voltage $V_{sample}$ and the voltage $V_{ref}$) is applied to the control unit 106. Additionally, in the control device 200B, the element electrically connected to the noninverting input terminal of the operational amplifier 262 and the element electrically connected to the inverting input terminal may be reversed.

The second shunt resistor 252 is used to stabilize the voltage $V_{sample}$ and the voltage $V_{analog}$ according to it when the load 132 is detached from the aerosol inhalation device 100 and reliably detect the detachment of the load 132.

In the following description, the switch Q1 is assumed to be in the OFF state, and the switch Q2 is assumed to be in the ON state. When the load 132 is attached, the voltage $V_{sample}$ applied to the noninverting input terminal of the operational amplifier 262 is a voltage obtained by dividing the voltage $V_{out}$ by the first shunt resistor 212 and the combined resistor (the electrical resistance value of the combined resistor is represented by R') of the second shunt resistor 252, the resistor 254, and the load 132. That is,

$$V_{sample} = \frac{R'}{R_{shunt1} + R'} \cdot V_{out} \qquad (4)$$

Here,

$$\frac{1}{R'} = \frac{1}{R_{shunt2}} + \frac{1}{R_{connector} + R_{HTR}}$$

$$\frac{1}{R'} = \frac{R_{shunt2} + R_{connector} + R_{HTR}}{R_{shunt2} \cdot (R_{connector} + R_{HTR})} \qquad (5)$$

$$R' = \frac{R_{shunt2} \cdot (R_{connector} + R_{HTR})}{R_{shunt2} + R_{connector} + R_{HTR}}$$

On the other hand, when the load 132 is detached, the voltage $V_{sample}$ applied to the noninverting input terminal of the operational amplifier 262 is a voltage obtained by dividing the voltage $V_{out}$ by the first shunt resistor 212 and the second shunt resistor 252. That is,

$$V_{sample} = \frac{R_{shunt2}}{R_{shunt1} + R_{shunt2}} \cdot V_{out} \qquad (6)$$

As described above, the first shunt resistor 212 and the second shunt resistor 252 have electrical resistance values sufficiently larger than that of the resistor 254 or the load 132. Hence, since R' is obviously different from $R_{shunt2}$, based on equations (5), the voltage $V_{sample}$ changes between a state in which the load 132 is attached and a state in which the load 132 is detached, as can be seen from equations (4) and (6). Hence, the voltage $V_{analog}$ according to the voltage $V_{sample}$ also changes between the state in which the load 132 is attached and the state in which the load 132 is detached. This allows the control unit 106 to detect attachment/detachment of the load 132 based on the applied voltage. If the second shunt resistor 252 is assumed to be absent, when the load 132 is detached, a path that starts from the power supply 110, passes through the first shunt resistor 212, and returns to the power supply 110 again does not form a closed circuit. Hence, the value of the voltage $V_{analog}$ becomes unstable. The control device 200B including the second shunt resistor 252 has such an advantage.

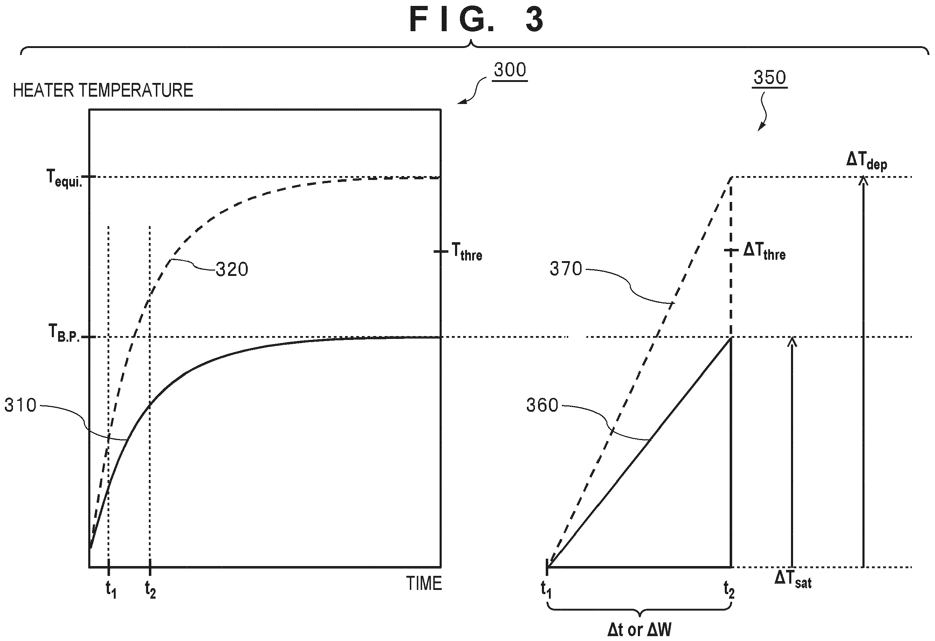

Fig. 3 shows a graph 300 schematically showing a time-series change (to be also referred to as a "temperature profile" hereinafter) in the temperature (to be also referred to as a "heater temperature" hereinafter) of the load 132 after the start of power feed to the load 132, and a temperature change 350 of the load 132 per predetermined time or predetermined supplied power.

In the graph 300, reference numeral 310 represents a schematic temperature profile of the load 132 when the remaining amount of the aerosol source in the holding portion 130 or the like is sufficient. $T_{B.P.}$ represents a boiling point or the like of the aerosol source. The temperature profile 310 shows that when the remaining amount of the aerosol source in the holding portion 130 or the like is sufficient, the temperature of the load 132 enters the steady state at the boiling point $T_{B.P.}$ or the like of the aerosol source or near the boiling point $T_{B.P.}$ or the like after the start of rising. It is considered that this is because, finally, almost all of the power supplied to the load 132 is consumed to atomize the aerosol source in the holding portion 130 or the like, and the increase in the temperature of the load 132 by the supplied power does not occur.

Note that the temperature profile 310 merely schematically represents the outline, and in fact, the temperature of the load 132 includes a local variation, and some transient change (not shown) may occur. These transient changes may be caused by a temperature localization that can temporarily occur in the load 132, or chattering that occurs in a sensor configured to detect the temperature itself of the load 132 or an electric parameter corresponding to the temperature of the load 132.

In the graph 300, reference numeral 320 represents a schematic temperature profile of the load 132 when the remaining amount of the aerosol source in the holding portion 130 or the like is not sufficient. The temperature profile 320 shows that when the remaining amount of the aerosol source in the holding portion 130 or the like is not sufficient, the temperature of the load 132 may enter the steady state at an equilibrium temperature $T_{equi.}$ higher than the boiling point $T_{B.P.}$ or the like of the aerosol source after the start of rising. It is considered that this is because, finally, temperature rising caused by the power applied to the load 132, temperature lowering caused by heat transfer to a substance (including a gas around the load 132 or a part of the structure of the aerosol inhalation device 100) near the load 132, and in some cases, temperature lowering caused by heat of vaporization of a small amount of aerosol source in the aerosol base material 116B or the holding portion 130 balance. Note that it was confirmed that if the remaining amount of the aerosol source in the holding portion 130 or the like is not sufficient, the load 132 sometimes enters the steady state at a different temperature in accordance with the remaining amount of the aerosol source in the aerosol base material 116B or the holding portion 130, the remaining amount in the aerosol source in the aerosol base material 116A (which can affect the supply speed of the aerosol source to the holding portion 130), the distribution of the aerosol source in the aerosol base material 116B or the holding portion 130, or the like. The equilibrium temperature $T_{equi.}$ is one of such temperatures, preferably, a temperature that is one of such temperatures and is not the highest temperature (the temperature when the remaining amount of the aerosol source in the aerosol base material 116B or the holding portion 130 is completely zero). It was also confirmed that if the remaining amount of the aerosol source in the holding portion or the like is not sufficient, in some cases, the temperature of the load 132 does not enter the steady state. Even at this time, the temperature of the load 132 reaches a temperature higher than the boiling point $T_{B.P.}$ or the like of the aerosol source.

Based on the above-described schematic temperature profile of the load 132 when the aerosol source in the holding portion 130 or the like is sufficient and that when the aerosol source is not sufficient, basically, it can be judged whether the remaining amount of the aerosol source in the holding portion 130 or the like is sufficient or not (that is, short or depleted) by determining whether the temperature of the load 132 exceeds a predetermined temperature threshold $T_{thre}$ ranging from the boiling point $T_{B.P.}$ or the like of the aerosol source (inclusive) to the equilibrium temperature $T_{equi.}$ (inclusive).

The temperature change 350 of the load 132 per predetermined time represents a temperature change of the load 132 per predetermined time $\Delta t$ from time t1 to time t2 in the graph 300. Reference numerals 360 and 370 correspond to a temperature change when the remaining amount of the aerosol source in the holding portion 130 or the like is sufficient and that when the remaining amount of the aerosol source is not sufficient, respectively. The temperature change 360 shows that when the remaining amount of the aerosol source in the holding portion 130 or the like is sufficient, the temperature of the load 132 rises only by $\Delta T_{sat}$ per predetermined time $\Delta t$. In addition, the temperature change 370 shows that when the remaining amount of the aerosol source in the holding portion 130 or the like is not sufficient, the temperature of the load 132 rises only by $\Delta T_{dep}$ larger than $\Delta T_{sat}$ per predetermined time $\Delta t$. Note that $\Delta T_{sat}$ and $\Delta T_{dep}$ change depending on the length of the predetermined time $\Delta t$, and even if the length of the predetermined time $\Delta t$ is fixed, $\Delta T_{sat}$ and $\Delta T_{dep}$ change when t1 (and t2) are changed. $\Delta T_{sat}$ and $\Delta T_{dep}$ may be considered as a maximum temperature change that can occur when t1 (and t2) are changed in the predetermined time $\Delta t$ with a certain length.

Based on the above-described temperature change of the load 132 per predetermined time when the aerosol source in the holding portion 130 or the like is sufficient and that when the aerosol source is not sufficient, basically, it can be judged whether the remaining amount of the aerosol source in the holding portion or the like is sufficient or not (that is, short or depleted) by determining whether the temperature change per predetermined time $\Delta t$ exceeds a predetermined temperature change threshold $\Delta T_{thre}$ ranging from $\Delta T_{sat}$ (inclusive) to $\Delta T_{dep}$ (inclusive).

Note that it is understood that it can be judged, using the temperature change of the load 132 per predetermined power $\Delta W$ supplied to the load 132 in place of the temperature change per predetermined time $\Delta t$, whether the remaining amount of the aerosol source in the holding portion or the like is sufficient or not.

To acquire the temperature $T_{HTR}$ of the load 132 by equation (3), a specific value such as the room temperature (for example, 25°C) needs to be defined as the reference temperature $T_{ref.}$

However, even if power supply to the heater for aerosol generation ends, a long time is needed until the temperature of the heater returns to the room temperature. In this case, the temperature of the heater measured after the end of power supply can have an error with respect to the reference temperature (for example, the room temperature) defined in advance. In particular, such an error can be large in a situation in which the user continuously performs inhalation using the aerosol inhalation device 100. When an error $\varepsilon$ is taken into consideration, equation (3) can be rewritten as

$$T_{HTR} = \frac{R_{HTR} - R_{ref}}{\alpha_{TCR}} + T_{ref} \pm \epsilon \qquad (7)$$

Hence, if the error $\varepsilon$ has a large positive value, the calculated temperature $T_{HTR}$ of the load 132 becomes higher than a truth value. Conversely, if the error $\varepsilon$ has a large negative value, the calculated temperature $T_{HTR}$ of the load 132 becomes lower than a truth value.

Based on the above-described findings, the present inventors arrived at an aerosol inhalation device, which can correctly estimate the temperature of a load and/or correctly detect the remaining amount of an aerosol source.

Processing for measuring the temperature of the load 132 and judging an occurrence of depletion or shortage of the aerosol source according to the embodiment of the present invention will be described below. As for the processing to be described below, it is assumed that the control unit 106 executes all steps. Note that some steps may be executed by another component of the aerosol inhalation device 100.

Figs. 4A and 4B are flowcharts of processing of measuring the temperature of the load 132 and determining depletion or shortage of the aerosol source according to the embodiment of the present invention. Processing 400 is repeated during the operation of the aerosol inhalation device 100.

In step 410, the control unit 106 determines whether a first condition and a second condition are satisfied. Upon determining that the first condition and the second condition are satisfied ("YES" in step 410), the process advances to step 420. Otherwise ("NO" in step 410), the process returns to the point before step 410. The first condition and the second condition will be described later.

In subsequent step 450 to be described later, a signal used to set the switch Q1 in the ON state to atomize the aerosol source is transmitted. If it is determined in step 410 that at least one of the first condition and the second condition is not satisfied, the process does not advance to step 450. Hence, setting the switch Q 1 in the ON state is inhibited.

In step 420, the control unit 106 determines whether an aerosol generation request is detected. Upon detecting the aerosol generation request ("YES" in step 420), the process advances to step 430. Otherwise ("NO" in step 420), the process returns to the point before step 420.

In step 420, upon detecting the start of inhalation by the user based on information obtained from, for example, a pressure sensor, a flow velocity sensor, a flow rate sensor, or the like, the control unit 106 may determine that the aerosol generation request is detected. More specifically, for example, if the output value (that is, the pressure) of the pressure sensor is smaller than a predetermined threshold, the control unit 106 can determine that the start of inhalation by the user is detected. Alternatively, for example, if the output value (that is, the flow velocity or the flow rate) of the flow velocity sensor or the flow rate sensor is larger than a predetermined threshold, the control unit 106 can determine that the start of inhalation by the user is detected. Since this determination method enables aerosol generation according to user's feeling, the flow velocity sensor or the flow rate sensor is particularly preferable. Alternatively, if the output values of these sensors start continuously changing, the control unit 106 may determine that the start of inhalation by the user is detected. Otherwise, based on pressing of a button used to start aerosol generation, or the like, the control unit 106 may determine that the start of inhalation by the user is detected. Alternatively, based on both the information obtained from the pressure sensor, the flow velocity sensor, or the flow rate sensor and pressing of the button, the control unit 106 may determine that the start of inhalation by the user is detected.

In step 430, the control unit 106 determines whether the value of a counter is equal to or smaller than a predetermined counter threshold. If the counter is equal to or less than the predetermined counter threshold ("YES" in step 430), the process advances to step 440. Otherwise ("NO" in step 430), the process advances to step 464 shown in Fig. 4B to be described later. Here, the predetermined counter threshold can be a predetermined value of 1 or more.

In step 440, the control unit determines whether the output of the temperature sensor 113 can be regarded as deviated from the actual temperature of the load 132. If the output of the temperature sensor 113 can be regarded as deviated from the actual temperature of the load 132 ("YES" in step 440), the process advances to step 448. Otherwise ("NO" in step 440), the process advances to step 442.

Fig. 5 is a graph showing a time-rate change in the temperature of the load 132 after the stop of power feed to the load 132. The abscissa of a graph 500 represents time after the stop of power feed to the load 132, and the ordinate represents the temperature of the load 132. Reference numeral 510 represents a plot showing an exemplary temperature change of the load 132 when the remaining amount of the aerosol source is sufficient. Reference numeral 520 represents three plots showing exemplary temperature changes of the load 132 when the remaining amount of the aerosol source is not sufficient.

As is understood from the graph 500, the temperature of the load 132 does not return to a room temperature $T_{R.T.}$ unless a long time (for example, 22 sec) elapses from the stop of power feed to the load 132. Hence, if next inhalation starts before the elapse of a sufficient time from the stop of power feed to the load 132, the heater temperature at the start of inhalation can greatly deviate from the room temperature. On the other hand, if a time to some extent (for example, 10 sec) elapses from the stop of power feed to the load 132, the magnitude of the deviation ($T'_{R.T.} - T_{R.T.}$) maintains a relatively small value, as can be understood.

Hence, as an example, in step 440, if the time elapsed from the stop of preceding power feed to the load 132 is shorter than a predetermined time (for example, 10 sec), the control unit 106 may regard the output of the temperature sensor (second sensor) 113 as deviated from the actual temperature of the load 132. On the other hand, if the elapsed time is equal to or longer than the predetermined time (for example, 10 sec), the control unit 106 may regard the output of the temperature sensor (second sensor) 113 as not deviated from the actual temperature of the load 132. That is, only when the time elapsed from the end of preceding power feed to the load 132 for aerosol generation is equal to or longer than a predetermined time, the process advances to step 442 to acquire the current output value of the second sensor 113, which is used in place of the previously acquired output value of the second sensor 113. This can also be expressed that only when a condition to judge that the difference between the temperature of the load 132 and the output value of the second sensor 113 is less than a threshold is satisfied, the current output value of the second sensor 113, which is used in place of the previously acquired output value of the second sensor 113, is acquired. This can also be expressed that only when a condition to judge that the temperature of the load 132 and the output value of the second sensor 113 almost equal is satisfied, the current output value of the second sensor 113, which is used in place of the previously acquired output value of the second sensor 113, is acquired. This can also be expressed that only when a condition to judge that the temperature of the load 132 and the output value of the second sensor 113 have a correlation is satisfied, the current output value of the second sensor 113, which is used in place of the previously acquired output value of the second sensor 113, is acquired. By these features, the reference temperature used in step 455 to be described later can more correctly be acquired, and the temperature of the load 132 can more correctly be calculated.

In step 442, the control unit 106 transmits a signal used to set the switch Q2 in the ON state to acquire the resistance value of the load 132.

In step 443, the control unit 106 acquires the resistance value of the load 132 as $R_2$ based on the above-described principle using equation (1), and at least temporarily stores it in the memory 114.

In step 444, the control unit 106 acquires the output of the temperature sensor 113 as a current temperature $T_2$ of the load 132, and at least temporarily stores it in the memory 114.

As described above, according to this embodiment, when inhalation is detected by the pressure sensor that detects inhalation by the user ("YES" in step 420), the control unit 106 acquires the output value of the first sensor 112 and the output value of the second sensor 113 before the start (step 450 to be described later) of power feed to the load for aerosol generation. The acquired values are used for the process of step 455 to be described later, and contributes to correct calculation of the temperature of the load 132.

In step 445, the control unit 106 transmits a signal used to set the switch Q2 in the OFF state.

In step 446, the control unit 106 substitutes the resistance value $R_2$ acquired in step 443 into a variable representing the reference resistance value $R_{ref}$ to calculate the temperature of the load 132 in step 455 to be described later.

In step 447, the control unit 106 substitutes the temperature $T_2$ acquired in step 444 into a variable representing the reference temperature $T_{ref}$ to calculate the temperature of the load 132 in step 455 to be described later.

On the other hand, in step 448, the control unit 106 substitutes one of the resistance values $R_2$ acquired in step 443 before the immediately preceding step or the value of a resistance value $R_1$ of the load 132 acquired at the time of exchange of the cartridge 104A to be described later into the variable representing the reference resistance value $R_{ref}$.

In step 449, the control unit 106 substitutes one of the temperatures $T_2$ acquired in step 444 before the immediately preceding step or the value of a temperature $T_1$ of the load 132 acquired at the time of exchange of the cartridge 104A to be described later into the variable representing the reference temperature $T_{ref}$.

In this way, the actually measured resistance value and temperature are stored as the reference resistance value and the reference temperature, respectively, thereby improving the accuracy of the temperature $T_{HTR}$ of the load 132 calculated by an equation used in step 455 to be described later.

In step 450, the control unit 106 transmits a signal used to set the switch Q1 in the ON state to atomize the aerosol source.

In step 451, the control unit 106 transmits a signal used to set the switch Q2 in the ON state to acquire the resistance value of the load 132.

In step 452, the control unit 106 transmits a signal used to set the switch Q1 in the OFF state to accurately acquire the resistance value of the load 132. It does not matter which of steps 451 and 452 is executed first, and the steps may be executed simultaneously. If a delay from the transmission of the signal to the switch to the actual change of the state is taken into consideration, step 451 is preferably performed before step 452.

In step 453, the control unit 106 acquires the resistance value of the load 132 as $R_3$ based on the above-described principle using equation (1).

In step 454, the control unit 106 transmits a signal used to set the switch Q2 in the OFF state.

In step 455, the control unit 106 acquires the temperature $T_{HTR}$ of the load 132 based on the above-described principle using equation (3). In this embodiment, $T_{ref}$ is not a value always fixed to the room temperature (for example, 25°C), and can flexibly be set in accordance with the situation by introducing steps 440, 444, 447, and 449 and step 632 and the like to be described later. Hence, in step 455, the temperature of the load 132 can accurately be calculated.

In step 460, the control unit 106 adds the temperature $T_{HTR}$ of the load 132 acquired in step 455 to a list that is a data structure, and makes the temperature referable later. Here, the list is merely an example of the data structure, and in step 460, an arbitrary data structure capable of holding a plurality of data such as an array may be used. Note that unless it is judged in step 470 to be described later that the process should advance to step 480, the process of step 460 is executed a plurality of times. If step 460 is executed a plurality of times, the temperature $T_{HTR}$ of the load 132 in the data structure is not overwritten but added as many as the number of times of execution of the process of step 460.

In step 462, the control unit 106 determines whether the temperature $T_{HTR}$ of the load 132 acquired in step 455 is lower than a predetermined first threshold. If the temperature $T_{HTR}$ of the load 132 is lower than the first threshold ("YES" in step 462), the process advances to step 470. Otherwise ("NO" in step 462), the process advances to step 464. The first threshold is preferably a temperature at which depletion of the aerosol source is strongly assumed if the temperature of the load 132 exceeds it. For example, the first threshold is 300°C. The process of step 462 is performed using the accurate temperature of the load 132 calculated in step 455. Hence, according to this embodiment, it is possible to accurately determine whether the aerosol source is depleted or short.

In step 464, the control unit 106 inhibits the switch Q1 from changing to the ON state. In step 464, the control unit 106 may set, in the memory 114, a flag concerning the first condition used in step 410. This flag may be canceled when the cartridge 104A is exchanged. That is, in this example, the first condition is that the cartridge 104A is exchanged. Until the flag is canceled (that is, until the cartridge 104A is exchanged), the first condition is not satisfied, and the determination in step 410 ends with "NO". In other words, unless the flag is canceled, the determination in step 410 never ends with "YES".

In step 466, the control unit 106 makes a predetermined notification on a UI (user interface) on the notification unit 108. This notification may be a notification representing that the cartridge 104A should be exchanged.

In step 470, the control unit 106 determines whether the aerosol generation request has ended. Upon determining that the aerosol generation request has ended ("YES" in step 470), the process advances to step 480. Otherwise ("NO" in step 470), the process returns to step 450. Upon detecting the end of inhalation by the user based on information obtained from, for example, a pressure sensor, a flow velocity sensor, a flow rate sensor, or the like, the control unit 106 may determine that the aerosol generation request has ended. Here, for example, if the output value (that is, the pressure) of the pressure sensor exceeds a predetermined threshold, the control unit 106 can determine that the end of inhalation by the user is detected (in other words, aerosol generation is not requested). Alternatively, for example, if the output value (that is, the flow velocity or the flow rate) of the flow velocity sensor or the flow rate sensor is smaller than a predetermined threshold, the control unit 106 can determine that the end of inhalation by the user is detected (in other words, aerosol generation is not requested). The predetermined threshold may be larger than the threshold in step 420, equal to the threshold, or smaller than the threshold. Otherwise, based on release of a button used to start aerosol generation, or the like, the control unit 106 may determine that the end of inhalation by the user is detected (in other words, aerosol generation is not requested). Alternatively, if a predetermined condition that, for example, a predetermined time has elapsed from the pressing of the button used to start aerosol generation is satisfied, the control unit 106 may determine that the end of inhalation by the user is detected (in other words, aerosol generation is not requested).

In step 480, the control unit 106 determines whether the maximum value in the list that holds one or more temperatures $T_{HTR}$ of the load 132 is smaller than a predetermined second threshold. If the maximum value is smaller than the second threshold ("YES" in step 480), the process advances to step 488. Otherwise ("NO" in step 480), the process advances to step 482. The second threshold is preferably a temperature at which depletion of the aerosol source is assumed when the temperature of the load 132 exceeds it, but at which there is also a possibility of temporary shortage of the aerosol source in the holding portion 130 due to, for example, a delay of aerosol source supply from the storage unit 116A. Hence, the second threshold may be smaller than the first threshold and is, for example, 250°C. The process of step 480 is performed using the accurate temperature of the load 132 calculated in step 455. Hence, according to this embodiment, it is possible to accurately determine whether the aerosol source is depleted or short.

In step 482, the control unit 106 temporarily inhibits the switch Q1 from changing to the ON state. This step may be a step of setting a flag concerning the second condition used in step 410 in the memory 114. This flag can be canceled when a predetermined time has elapsed from setting of the flag. That is, in this example, the second condition is that a predetermined time elapses from setting of the flag. Until the flag is canceled (that is, until a predetermined time has elapsed from setting of the flag), the second condition is not satisfied, and the determination in step 410 temporarily ends with "NO". In other words, unless the flag is canceled, the determination in step 410 never ends with "YES". Note that the predetermined time can be 10 sec or more, for example, 11 sec.

In step 484, the control unit 106 makes a predetermined notification on the UI on the notification unit 108. This notification can be a notification that promotes the user to wait for inhalation of the aerosol for a while.

In step 486, the control unit 106 increments the counter (for example, the counter is incremented by one).

In step 488, the control unit 106 initializes the counter and the list. By this step, the counter may be 0, and the list may be made empty.

In this embodiment, in step 480 after the aerosol generation request has ended, the temperature $T_{HTR}$ of the load 132 is compared with the second threshold. In place of this embodiment, the temperature $T_{HTR}$ of the load 132 may be compared with the second threshold before the aerosol generation request ends. If it is judged that the temperature $T_{HTR}$ of the load 132 is equal to or higher than the second threshold, comparison between the second threshold and the temperature $T_{HTR}$ of the load 132 need not be performed any more until the aerosol generation request ends.

Fig. 6 is a flowchart of processing 600 performed simultaneously with or in parallel to the processing 400 shown in Figs. 4A and 4B.

In step 610, the control unit 106 determines whether connection or exchange of the cartridge 104A is detected. A detailed method of detecting connection or exchange of the cartridge 104A has already been described in the explanation of Figs. 2A and 2B. If connection of the cartridge 104A is detected ("YES" in step 610), the process advances to step 620. Otherwise, the process returns to the point before step 610.

If the cartridge 104A is attached to the main body 102, the electronic circuit included in the cartridge 104A is electrically connected to the electronic circuit of the main body 102 via at least two terminals included in the main body 102.

In step 620, the control unit 106 transmits a signal used to set the switch Q2 in the ON state to acquire the resistance value of the load 132.

In step 630, the control unit 106 acquires the resistance value of the load 132 as $R_1$ based on the above-described principle using equation (1), and at least temporarily stores it in the memory 114.

In step 632, the control unit 106 acquires the output of the temperature sensor 113 as a current temperature $T_1$ of the load 132, and at least temporarily stores it in the memory 114.

As described above, according to this embodiment, when the first unit 104A is attached to the second unit 102, the control unit 106 acquires the output value of the first sensor 112 and the output value of the second sensor 113 before the start (step 450) of power feed to the load 132 for aerosol generation. The acquired values are used in steps 448 and 449, and contributes to correct calculation of the temperature of the load 132 in step 455. The acquisition of the resistance value $R_1$ in step 630 and the acquisition of the current temperature $T_1$ in step 632 are performed when the first unit 104A is attached to the second unit 102. The above-described acquisition of the resistance value $R_2$ in step 446 and the acquisition of the current temperature $T_2$ in step 447 are performed when the first unit 104A is attached to the second unit 102, and after that, the aerosol generation request is detected. That is, the resistance value $R_1$ and the current temperature $T_1$ are acquired as an early timing on the time base as compared to the resistance value $R_2$ and the current temperature $T_2$.

In step 640, the control unit 106 transmits a signal used to set the switch Q2 in the OFF state.

In step 650, the control unit 106 initializes the above-described counter and list used in the processing 400.

As can be understood from the explanation concerning Figs. 4A to 6, according to this embodiment, the control unit 106 calculates the temperature of the load 132 and/or judges whether the aerosol source is depleted or short based on the output value (a value concerning the electrical resistance value of the load 132 or the electrical resistance value measured in step 443 or 630) of the first sensor 112 and the output value (a temperature that is measured in step 444 or 632 and can be regarded as the temperature of the load 132) of the second sensor 113 before the start (step 450) of power feed to the load 132 for aerosol generation and the output value (a value concerning the electrical resistance value of the load 132 or the electrical resistance value measured in step 453) of the first sensor 112 after the start (step 450) of power feed to the load 132 for aerosol generation. When not a fixed value (for example, 25°C) but an actually measured temperature is used as the reference temperature of the load 132, the temperature of the load 132 can more correctly be calculated, and it can more correctly be judged whether the aerosol source is depleted or short.

Additionally, as can be understood from the explanation concerning Figs. 4A to 6, according to this embodiment, the control unit 106 acquires the output value of the first sensor 112 and the output value of the second sensor 113 before the start (step 450) of power feed to the load 132 for aerosol generation at a plurality of timings (for example, steps 630 and 632 after detection of connection of the cartridge 104A, steps 443 and 444 after detection of the preceding aerosol generation request, and steps 443 and 444 after detection of the current aerosol generation request). This makes it possible to selectively use the values to be used to calculate the temperature of the load 132 in step 455 based on the determination result of step 440 and the like and appropriately calculate the temperature of the load 132 in accordance with the situation.

Figs. 7-1 and 7-2 show a flowchart of processing corresponding to Figs. 4A-1 and 4A-2 in an arrangement using the thermistor 113A configured to detect the temperature of the power supply 110 to acquire the temperature of the load 132 according to the embodiment of the present invention.

The processes of steps 710 to 740 are the same as the processes of steps 410 to 440, and a description thereof will be omitted.

If the output of the temperature sensor (here, the thermistor 113A) can be regarded as deviated from the actual temperature of the load 132 ("YES" in step 740), the process advances to step 748. Otherwise ("NO" in step 740), the process advances to step 741.

As shown in Fig. 7-1, before the process to step 742 corresponding to step 442, in step 741, the control unit 106 acquires the output value of the thermistor 113A as the current temperature $T_2$ of the load 132, and at least temporarily stores it in the memory 114.

That is, according to this embodiment, before the start of power feed to the load 132 for aerosol generation, the control unit 106 acquires the output value of the thermistor (second sensor) 113A before the output value of the first sensor 112. By this feature, it is possible to prevent the temperature to be detected by the thermistor 113A from being raised by heat generation of the power supply 110 itself, which is caused by discharge of the power supply 110. It is therefore possible to more correctly acquire the temperature of the load 132 using the thermistor 113A.

Processing from step 742 is the same as the processing from step 442 in Fig. 4A-1 without step 444, and a description thereof will be omitted.

Fig. 8 is a flowchart of processing corresponding to Fig. 6 in the arrangement using the thermistor 113A configured to detect the temperature of the power supply 110 to measure the temperature of the load 132 according to the embodiment of the present invention.

The process of step 810 is the same as the process of step 610, and a description thereof will be omitted.

As shown in Fig. 8, before the process of step 820 corresponding to step 620, in step 812, the control unit 106 acquires the output value of the thermistor 113A as the current temperature $T_1$ of the load 132, and at least temporarily stores it in the memory 114.

That is, according to this embodiment, before the start of power feed to the load 132 for aerosol generation, the control unit 106 acquires the output value of the thermistor (second sensor) 113A before the output value of the first sensor 112. By this feature, it is possible to prevent the temperature to be detected by the thermistor 113A from being raised by heat generation of the power supply 110 itself, which is caused by discharge of the power supply 110. It is therefore possible to more correctly acquire the temperature of the load 132 using the thermistor 113A.

Processing from step 820 is the same as the processing from step 620 in Fig. 6 without step 632, and a description thereof will be omitted.

As can be understood from Figs. 7 and 8, according to this embodiment, if the second sensor 113 is the thermistor 113A configured to detect the temperature of the power supply 110, the control unit 106 acquires the output value of the first sensor 112 and the output value of the second sensor 113 before the start (step 750) of power feed to the load 132 for aerosol generation at least at the first timing (for example, steps 812 and 830 after detection of connection of the cartridge 104A or steps 741 and 743 after detection of the preceding aerosol generation request) and the second timing (for example, steps 741 and 743 after detection of the current aerosol generation request). Note that the first timing is earlier than the second timing on the time base. If it is judged that the temperature of the load 132 and the output value of the second sensor 113 do not have a predetermined relationship (for example, "YES" in step 740), the control unit 106 uses the output value of the first sensor 112 and the output value of the second sensor 113 acquired at the first timing to calculate the temperature of the load 132 and/or judge whether the aerosol source is depleted or short. Additionally, if it is judged that the temperature of the load 132 and the output value of the second sensor 113 have a predetermined relationship (for example, "NO" in step 740), the control unit 106 uses the output value of the first sensor 112 and the output value of the second sensor 113 acquired at the second timing to calculate the temperature of the load 132 and/or judge whether the aerosol source is depleted or short. This makes it possible to selectively use the acquired values to be used to calculate the temperature of the load 132 in step 755 based on the determination result of step 740 and the like and appropriately calculate the temperature of the load 132 in accordance with the situation.

In the description made above, the embodiment of the present invention has been described as an aerosol inhalation device.

While the embodiments of the present invention have been described above, it is to be understood that the embodiments are merely examples, and do not limit the scope of the present invention. It should be understood that modifications, additions, improvements, and the like of the embodiments can appropriately be made without departing from the scope of the present invention. The scope of the present invention should not be limited by any of the above-described embodiments, and should be limited only by the appended claims.

Reference Signs List

100...aerosol inhalation device, 102...second unit, 104A, 104B...first unit, 106...control unit, 108...notification unit, 110...power supply, 112...first sensor, 113...second sensor, 114...memory, 116A...storage unit, 116B...aerosol base material, 118A, 118B...atomization unit, 120...air intake channel, 121...aerosol channel, 122...mouthpiece portion, 130...holding portion, 132...load, 134...circuit, 200A, 200B...control device, 202...first circuit, 204...second circuit, 208...conversion unit, 212...first shunt resistor, 224...pressure sensor, 226...absolute pressure sensor, 230...calibration unit, 252...second shunt resistor, 256, 258...connection terminal, 262...operational amplifier, 310, 320...temperature profile

**Claims**

1. An aerosol inhalation device comprising:

   a first sensor (112) configured to output one of a value concerning an electrical resistance value of a load (132) and the electrical resistance value, the load (132) being configured to heat an aerosol source and having a correlation between a temperature and the electrical resistance value; and
   a power supply (110),
   **characterized by** comprising:

   a second sensor (113) comprising a thermistor configured to detect a temperature of the power supply (110), and
   a control unit (106) configured to calculate the temperature of the load (132) and/or judge whether the aerosol source is depleted based on an output value of the first sensor (112) and an output value of the second sensor (113), which are obtained before a start of power feed to the load for aerosol generation, and an output value of the first sensor (112) which is obtained after the start of power feed to the load (132) for aerosol generation;
   a first unit (104A) including the load (132); and
   a second unit (102) including the first sensor (112), the second sensor (113), the power supply (110) and the control unit (106),
   wherein the first unit(104A) is detachable from the second unit (102).

2. The device according to claim 1, wherein the control unit (106) is configured to acquire the output value of the second sensor (113) before the output value of the first sensor (112) and before the start of power feed to the load (132) for aerosol generation.

3. The device according to claim 1 or 2, wherein the control unit (106) is configured to acquire a current output value of the second sensor (113), which is used in place of a previously acquired output value of the second sensor (113), before the start of power feed to the load (113) for aerosol generation only if a condition to judge that a difference between the temperature of the load (132) and the output value of the second sensor (113) is less than a threshold is satisfied.

4. The device according to claim 1 or 2, wherein the control unit (106) is configured to acquire a current output value of the second sensor (113), which is used in place of a previously acquired output value of the second sensor (113), before the start of power feed to the load for aerosol generation only if a condition to judge that the temperature of the load (132) and the output value of the second sensor are almost equal is satisfied.

5. The device according to claim 1 or 2, wherein the control unit (106) is configured to acquire a current output value of the second sensor (113), which is used in place of a previously acquired output value of the second sensor (113), before the start of power feed to the load for aerosol generation only if a condition to judge that the temperature of the load (132) and the output value of the second sensor (113) have a correlation is satisfied.

6. The device according to claims 1 or 2, wherein the control unit (106) is configured to acquire a current output value of the second sensor (113), which is used in place of a previously acquired output value of the second sensor (113), before the start of power feed to the load for aerosol generation only if a time elapsed from an end of preceding power feed to the load for aerosol generation is not less than a predetermined time.

**7.** The device according to claim 1,
wherein the control unit (106) is configured to acquire the output value of the first sensor (112) and the output value of the second sensor (113) before the start of power feed to the load for aerosol generation when the first unit (104A) is attached to the second unit (102).

**8.** The device according to claim 1, further comprising a third sensor configured to detect inhalation by a user, wherein the control unit (106) is configured to acquire the output value of the first sensor (112) and the output value of the second sensor (113) before the start of power feed to the load for aerosol generation when the inhalation is detected by the third sensor.

**9.** The device according to claim 1, wherein the control unit (106) is configured to acquire, at a plurality of timings, the output value of the first sensor (112) and the output value of the second sensor (113) before the start of power feed to the load (132) for aerosol generation.

**10.** The device according to claim 1, wherein

the control unit (106) is configured to
acquire, at least at a first timing and a second timing, the output value of the first sensor (112) and the output value of the second sensor (113) before the start of power feed to the load (132) for aerosol generation,
upon judging that the temperature of the load (132) and the output value of the second sensor (113) do not have a predetermined relationship, use the output value of the first sensor (112) and the output value of the second sensor (113), which are acquired at the first timing, to calculate the temperature of the load (132) and/or judge whether the aerosol source is depleted, and
upon judging that the temperature of the load (132) and the output value of the second sensor (113) have a predetermined relationship, use the output value of the first sensor (112) and the output value of the second sensor (113), which are acquired at the second timing, to calculate the temperature of the load (132) and/or judge whether the aerosol source is depleted.

**11.** The device according to claim 10, wherein the first timing is earlier than the second timing on a time base.

**12.** The device according to claim 10,
wherein the first timing is a timing at which the first unit is attached to the second unit.

**13.** The device according to claim 11 or 12, further comprising a third sensor configured to detect inhalation by a user, wherein the second timing is a timing at which the inhalation is detected by the third sensor.


**Patentansprüche**

**1.** Aerosol-Inhalationsvorrichtung, die Folgendes umfasst:

einen ersten Sensor (112), der so konfiguriert ist, dass er einen von einem Wert, der einen elektrischen Widerstandswert einer Last (132) betrifft, und dem elektrischen Widerstandswert ausgibt, wobei die Last (132) so konfiguriert ist, dass sie eine Aerosolquelle erwärmt und eine Korrelation zwischen einer Temperatur und dem elektrischen Widerstandswert aufweist; und
eine Stromversorgung (110),
**dadurch gekennzeichnet, dass** sie Folgendes umfasst:

einen zweiten Sensor (113), der einen Thermistor umfasst, der so konfiguriert ist, dass er eine Temperatur der Stromversorgung (110) erkennt, und
eine Steuereinheit (106), die so konfiguriert ist, dass sie die Temperatur der Last (132) berechnet und/oder beurteilt, ob die Aerosolquelle erschöpft ist, basierend auf einem Ausgabewert des ersten Sensors (112) und einem Ausgabewert des zweiten Sensors (113), die vor einem Beginn der Energiezufuhr zu der Last zur Aerosolerzeugung erhalten werden, und einem Ausgabewert des ersten Sensors (112), der nach dem Beginn der Energiezufuhr zu der Last (132) zur Aerosolerzeugung erhalten wird;
eine erste Einheit (104A), die die Last (132) einschließt; und
eine zweite Einheit (102), die den ersten Sensor (112), den zweiten Sensor (113), die Stromversorgung (110) und die Steuereinheit (106) einschließt,

wobei die erste Einheit (104A) von der zweiten Einheit (102) abnehmbar ist.

2. Vorrichtung nach Anspruch 1, wobei die Steuereinheit (106) so konfiguriert ist, dass sie den Ausgabewert des zweiten Sensors (113) vor dem Ausgabewert des ersten Sensors (112) und vor dem Beginn der Energiezufuhr zur Last (132) zur Aerosolerzeugung erfasst.

3. Vorrichtung nach Anspruch 1 oder 2, wobei die Steuereinheit (106) so konfiguriert ist, dass sie einen aktuellen Ausgabewert des zweiten Sensors (113), der anstelle eines zuvor erfassten Ausgabewerts des zweiten Sensors (113) verwendet wird, vor dem Beginn der Energiezufuhr zu der Last (113) zur Aerosolerzeugung nur dann erfasst, wenn eine Bedingung zur Beurteilung, dass eine Differenz zwischen der Temperatur der Last (132) und dem Ausgabewert des zweiten Sensors (113) kleiner als ein Schwellenwert ist, erfüllt ist.

4. Vorrichtung nach Anspruch 1 oder 2, wobei die Steuereinheit (106) so konfiguriert ist, dass sie einen aktuellen Ausgabewert des zweiten Sensors (113), der anstelle eines zuvor erfassten Ausgabewerts des zweiten Sensors (113) verwendet wird, vor dem Beginn der Energiezufuhr zu der Last zur Aerosolerzeugung nur dann erfasst, wenn eine Bedingung zur Beurteilung, dass die Temperatur der Last (132) und der Ausgabewert des zweiten Sensors nahezu gleich sind, erfüllt ist.

5. Vorrichtung nach Anspruch 1 oder 2, wobei die Steuereinheit (106) so konfiguriert ist, dass sie einen aktuellen Ausgabewert des zweiten Sensors (113), der anstelle eines zuvor erfassten Ausgabewerts des zweiten Sensors (113) verwendet wird, vor dem Beginn der Energiezufuhr zu der Last zur Aerosolerzeugung nur dann erfasst, wenn eine Bedingung zur Beurteilung, dass die Temperatur der Last (132) und der Ausgabewert des zweiten Sensors (113) eine Korrelation aufweisen, erfüllt ist.

6. Vorrichtung nach Anspruch 1 oder 2, wobei die Steuereinheit (106) so konfiguriert ist, dass sie einen aktuellen Ausgabewert des zweiten Sensors (113), der anstelle eines zuvor erfassten Ausgabewerts des zweiten Sensors (113) verwendet wird, vor dem Beginn der Energiezufuhr zu der Last zur Aerosolerzeugung nur dann erfasst, wenn eine Zeit, die von einem Ende einer vorangegangenen Energiezufuhr zu der Last zur Aerosolerzeugung verstrichen ist, nicht kleiner als eine vorbestimmte Zeit ist.

7. Vorrichtung nach Anspruch 1,
   wobei die Steuereinheit (106) so konfiguriert ist, dass sie den Ausgabewert des ersten Sensors (112) und den Ausgabewert des zweiten Sensors (113) vor dem Beginn der Energiezufuhr zur Last zur Aerosolerzeugung erfasst, wenn die erste Einheit (104A) mit der zweiten Einheit (102) verbunden ist.

8. Vorrichtung nach Anspruch 1, die weiter einen dritten Sensor umfasst, der so konfiguriert ist, dass er die Inhalation durch einen Benutzer erkennt,
   wobei die Steuereinheit (106) so konfiguriert ist, dass sie den Ausgabewert des ersten Sensors (112) und den Ausgabewert des zweiten Sensors (113) vor dem Beginn der Energiezufuhr zur Last zur Aerosolerzeugung erfasst, wenn die Inhalation durch den dritten Sensor erkannt wird.

9. Vorrichtung nach Anspruch 1, wobei die Steuereinheit (106) so konfiguriert ist, dass sie zu einer Vielzahl von Zeitpunkten den Ausgabewert des ersten Sensors (112) und den Ausgabewert des zweiten Sensors (113) vor dem Beginn der Energiezufuhr zu der Last (132) zur Aerosolerzeugung erfasst.

10. Vorrichtung nach Anspruch 1, wobei

    die Steuereinheit (106) konfiguriert ist zum
    Erfassen des Ausgabewerts des ersten Sensors (112) und des Ausgabewerts des zweiten Sensors (113) zumindest zu einem ersten Zeitpunkt und zu einem zweiten Zeitpunkt vor dem Beginn der Energiezufuhr zur Last (132) zur Aerosolerzeugung,
    bei der Beurteilung, dass die Temperatur der Last (132) und der Ausgabewert des zweiten Sensors (113) keine vorbestimmte Beziehung aufweisen, Verwenden des Ausgabewertes des ersten Sensors (112) und des Ausgabewertes des zweiten Sensors (113), die zum ersten Zeitpunkt erfasst werden, um die Temperatur der Last (132) zu berechnen und/oder zu beurteilen, ob die Aerosolquelle erschöpft ist, und
    bei der Beurteilung, dass die Temperatur der Last (132) und der Ausgabewert des zweiten Sensors (113) eine vorbestimmte Beziehung aufweisen, Verwenden des Ausgabewertes des ersten Sensors (112) und des Ausgabewertes des zweiten Sensors (113), die zum zweiten Zeitpunkt erfasst werden, um die Temperatur der Last (132) zu berechnen und/oder zu beurteilen, ob die Aerosolquelle erschöpft ist.

**11.** Vorrichtung nach Anspruch 10, wobei der erste Zeitpunkt auf einer Zeitbasis vor dem zweiten Zeitpunkt liegt.

**12.** Vorrichtung nach Anspruch 10,
wobei der erste Zeitpunkt ein Zeitpunkt ist, zu dem die erste Einheit an der zweiten Einheit befestigt ist.

**13.** Vorrichtung nach Anspruch 11 oder 12, die weiter einen dritten Sensor umfasst, der so konfiguriert ist, dass er die Inhalation durch einen Benutzer erkennt,
wobei der zweite Zeitpunkt ein Zeitpunkt ist, zu dem die Inhalation durch den dritten Sensor erkannt wird.

**Revendications**

**1.** Dispositif d'inhalation d'aérosol comprenant :

un premier capteur (112) configuré pour délivrer en sortie l'une parmi une valeur concernant une valeur de résistance électrique d'une charge (132) et la valeur de résistance électrique, la charge (132) étant configurée pour chauffer une source d'aérosol et présentant une corrélation entre une température et la valeur de résistance électrique ; et
une alimentation électrique (110),
**caractérisé en ce qu'**il comprend :

un deuxième capteur (113) comprenant une thermistance configurée pour détecter une température de l'alimentation électrique (110), et
une unité de commande (106) configurée pour calculer la température de la charge (132) et/ou juger si la source d'aérosol est épuisée sur la base d'une valeur de sortie du premier capteur (112) et d'une valeur de sortie du deuxième capteur (113), qui sont obtenues avant un début de l'alimentation électrique de la charge pour la génération d'aérosol, et une valeur de sortie du premier capteur (112) qui est obtenue après le début de l'alimentation électrique de la charge (132) pour la génération d'aérosol ;
une première unité (104A) incluant la charge (132) ; et
une seconde unité (102) incluant le premier capteur (112), le deuxième capteur (113), l'alimentation électrique (110) et l'unité de commande (106),
dans lequel la première unité (104A) est apte à être détachée de la seconde unité (102).

**2.** Dispositif selon la revendication 1, dans lequel l'unité de commande (106) est configurée pour acquérir la valeur de sortie du deuxième capteur (113) avant la valeur de sortie du premier capteur (112) et avant le début de l'alimentation électrique de la charge (132) pour la génération d'aérosol.

**3.** Dispositif selon la revendication 1 ou la revendication 2, dans lequel l'unité de commande (106) est configurée pour acquérir une valeur de sortie actuelle du deuxième capteur (113), qui est utilisée à la place d'une valeur de sortie du deuxième capteur (113) acquise précédemment, avant le début de l'alimentation électrique de la charge (113) pour la génération d'aérosol seulement si une condition pour juger qu'une différence entre la température de la charge (132) et la valeur de sortie du deuxième capteur (113) est inférieure à un seuil est remplie.

**4.** Dispositif selon la revendication 1 ou la revendication 2, dans lequel l'unité de commande (106) est configurée pour acquérir une valeur de sortie actuelle du deuxième capteur (113), qui est utilisée à la place d'une valeur de sortie du deuxième capteur (113) acquise précédemment, avant le début de l'alimentation électrique de la charge pour la génération d'aérosol seulement si une condition pour juger que la température de la charge (132) et la valeur de sortie du deuxième capteur sont presque égales est remplie.

**5.** Dispositif selon la revendication 1 ou la revendication 2, dans lequel l'unité de commande (106) est configurée pour acquérir une valeur de sortie actuelle du deuxième capteur (113), qui est utilisée à la place d'une valeur de sortie du deuxième capteur (113) acquise précédemment, avant le début de l'alimentation électrique de la charge pour la génération d'aérosol seulement si une condition pour juger que la température de la charge (132) et la valeur de sortie du deuxième capteur (113) ont une corrélation est remplie.

**6.** Dispositif selon la revendication 1 ou la revendication 2, dans lequel l'unité de commande (106) est configurée pour acquérir une valeur de sortie actuelle du deuxième capteur (113), qui est utilisée à la place d'une valeur de sortie du deuxième capteur (113) acquise précédemment, avant le début de l'alimentation électrique de la charge pour la génération d'aérosol seulement si un temps écoulé à partir d'une fin de l'alimentation électrique précédente de la charge pour la génération d'aérosol n'est pas inférieur à un temps prédéterminé.

7. Dispositif selon la revendication 1,
dans lequel l'unité de commande (106) est configurée pour acquérir la valeur de sortie du premier capteur (112) et la valeur de sortie du deuxième capteur (113) avant le début de l'alimentation électrique de la charge pour la génération d'aérosol lorsque la première unité (104A) est attachée à la seconde unité (102).

8. Dispositif selon la revendication 1, comprenant en outre un troisième capteur configuré pour détecter une inhalation par un utilisateur,
dans lequel l'unité de commande (106) est configurée pour acquérir la valeur de sortie du premier capteur (112) et la valeur de sortie du deuxième capteur (113) avant le début de l'alimentation électrique de la charge pour la génération d'aérosol lorsque l'inhalation est détectée par le troisième capteur.

9. Dispositif selon la revendication 1, dans lequel l'unité de commande (106) est configurée pour acquérir, à une pluralité de moments, la valeur de sortie du premier capteur (112) et la valeur de sortie du deuxième capteur (113) avant le début de l'alimentation électrique de la charge (132) pour la génération d'aérosol.

10. Dispositif selon la revendication 1, dans lequel

l'unité de commande (106) est configurée pour
acquérir, au moins à un premier moment et à un second moment, la valeur de sortie du premier capteur (112) et la valeur de sortie du deuxième capteur (113) avant le début de l'alimentation électrique de la charge (132) pour la génération d'aérosol,
lorsqu'il est jugé que la température de la charge (132) et la valeur de sortie du deuxième capteur (113) n'ont pas une relation prédéterminée, utiliser la valeur de sortie du premier capteur (112) et la valeur de sortie du deuxième capteur (113), qui sont acquises au premier moment, pour calculer la température de la charge (132) et/ou juger si la source d'aérosol est épuisée, et
lorsqu'il est jugé que la température de la charge (132) et la valeur de sortie du deuxième capteur (113) ont une relation prédéterminée, utiliser la valeur de sortie du premier capteur (112) et la valeur de sortie du deuxième capteur (113), qui sont acquises au second moment, pour calculer la température de la charge (132) et/ou juger si la source d'aérosol est épuisée.

11. Dispositif selon la revendication 10, dans lequel le premier moment est antérieur au second moment sur une base temporelle.

12. Dispositif selon la revendication 10,
dans lequel le premier moment est un moment auquel la première unité est attachée à la seconde unité.

13. Dispositif selon la revendication 11 ou la revendication 12, comprenant en outre un troisième capteur configuré pour détecter une inhalation par un utilisateur,
dans lequel le second moment est un moment auquel l'inhalation est détectée par le troisième capteur.

FIG. 1A

FIG. 1B

# FIG. 2A

EP 3 744 189 B1

F I G. 2B

**F I G. 3**

START

400

410

FIRST CONDITION AND SECOND CONDITION SATISFIED? — NO

YES

420

AEROSOL GENERATION REQUEST DETECTED? — NO

YES

430

COUNTER ≤ COUNTER THRESHOLD ? — NO → (A)

YES

440

CAN OUTPUT OF TEMPERATURE SENSOR BE REGARDED AS DEVIATED FROM ACTUAL TEMPERATURE OF LOAD ? — NO

YES

(C)

442
Q2 ON

443 — MEASURE RESISTANCE VALUE $R_2$ OF LOAD

444 — MEASURE CURRENT TEMPERATURE $T_2$

445 — Q2 OFF

446 — $R_{ref} = R_2$

447 — $T_{ref} = T_2$

(B)

# FIG. 4A-1

# F I G. 4A-2

400

(C)

| |
|---|
| $R_{ref} = R_1$ or $R_2$ |
~448

| |
|---|
| $T_{ref} = T_1$ or $T_2$ |
~449

(D) ──────────────────►◄────────────────── (B)

| |
|---|
| Q1 ON |
~450

| |
|---|
| Q2 ON |
~451

| |
|---|
| Q1 OFF |
~452

| |
|---|
| MEASURE RESISTANCE VALUE $R_3$ OF LOAD |
~453

| |
|---|
| Q2 OFF |
~454

| |
|---|
| $T_{HTR} = T_{ref} + \dfrac{R_3 - R_{ref}}{\alpha_{TCR}}$ |
~455

(E)

F I G. 4B

E

ADD $T_{HTR}$ TO LIST ⎯ 460

400

462

$T_{HTR}$ < FIRST THRESHOLD? ⎯ NO → A

YES

464 ⎯ INHIBIT ON OF Q1

466 ⎯ MAKE FIRST NOTIFICATION ON UI

F

470

AEROSOL GENERATION REQUEST ENDED? ⎯ NO → D

YES

480

MAXIMUM VALUE IN LIST < SECOND THRESHOLD? ⎯ NO

YES

488

INITIALIZE COUNTER AND LIST

482 ⎯ TEMPORARILY INHIBIT ON OF Q1

484 ⎯ MAKE SECOND NOTIFICATION ON UI

486 ⎯ INCREMENT COUNTER

F

END

EP 3 744 189 B1

# FIG. 5

# FIG. 6

600

START

610

CARTRIDGE CONNECTION DETECTED?

NO

YES

Q2 ON — 620

MEASURE RESISTANCE VALUE $R_1$ OF LOAD — 630

MEASURE CURRENT TEMPERATURE $T_1$ — 632

Q2 OFF — 640

INITIALIZE COUNTER AND LIST — 650

END

START

710

FIRST CONDITION AND
SECOND CONDITION
SATISFIED?

NO

700

YES

720

AEROSOL GENERATION
REQUEST DETECTED?

NO

YES

730

COUNTER ≤
COUNTER THRESHOLD
?

NO

(A)

YES

740

CAN OUTPUT OF
TEMPERATURE SENSOR BE
REGARDED AS DEVIATED
FROM ACTUAL TEMPERATURE
OF LOAD
?

NO

741 — MEASURE CURRENT
TEMPERATURE $T_2$

742 — Q2 ON

743 — MEASURE RESISTANCE
VALUE $R_2$ OF LOAD

745 — Q2 OFF

746 — $R_{ref} = R_2$

747 — $T_{ref} = T_2$

YES

(C)

(B)

# FIG. 7-1

# FIG. 7-2

700

$R_{ref} = R_1$ or $R_2$ ——— 748

$T_{ref} = T_1$ or $T_2$ ——— 749

Q1 ON ——— 750

Q2 ON ——— 751

Q1 OFF ——— 752

MEASURE RESISTANCE VALUE $R_3$ OF LOAD ——— 753

Q2 OFF ——— 754

$T_{HTR} = T_{ref} + \dfrac{R_3 - R_{ref}}{\alpha_{TCR}}$ ——— 755

# FIG. 8

800

START

810

CARTRIDGE CONNECTION DETECTED?

NO

YES

MEASURE CURRENT TEMPERATURE $T_1$ — 812

Q2 ON — 820

MEASURE RESISTANCE VALUE $R_1$ OF LOAD — 830

Q2 OFF — 840

INITIALIZE COUNTER AND LIST — 850

END

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017144374 A1
- WO 2017144191 A1
- WO 2018001746 A1

- WO 2012085203 A
- WO 2012085207 A
- WO 2017144191 A